(19) Europäisches Patentamt
European Patent Office
Office européen des brevets

(11) EP 2 395 041 A1

(12) **EUROPEAN PATENT APPLICATION**

(43) Date of publication:
**14.12.2011 Bulletin 2011/50**

(21) Application number: **10165502.5**

(22) Date of filing: **10.06.2010**

(51) Int Cl.:
***C08G 73/02*** *(2006.01)*
***C08G 69/26*** *(2006.01)*
***C08G 69/08*** *(2006.01)*
***A61K 47/34*** *(2006.01)*
***C12N 15/113*** *(2010.01)*
***A61K 48/00*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**
Designated Extension States:
**BA ME RS**

(71) Applicants:
- **F. Hoffmann-La Roche AG**
**4070 Basel (CH)**
- **Ludwig-Maximilians-Universität München**
**80359 München (DE)**

(72) Inventor: **The designation of the inventor has not yet been filed**

(74) Representative: **Küng, Peter et al**
**F. Hoffmann-La Roche AG**
**Patent Department**
**Grenzacherstrasse 124**
**4070 Basel (CH)**

(54) **Polymers for delivery of nucleic acids**

(57) Polyamide polymers containing defined oligo (alkylene amino) acids, methods for their production, their use as a delivery reagent for nucleic acids, pharmaceutical compositions containing said polyamide polymers, and uses thereof are provided.

EP 2 395 041 A1

## Description

The present invention relates to polyamide polymers (PAA) containing defined oligo (alkylene amino) acids (OAA), methods for their production, their use as a delivery reagent for nucleic acids, pharmaceutical compositions containing said polyamide polymers, and uses thereof.

In the recent years the application of nucleic acids has become a powerful approach in molecular medicine and diagnostics. Of particular interest is the specific targeting and delivery of double stranded RNA molecules (dsRNA) to and into target tissues and target cells. Double-stranded ribonucleic acid (dsRNA) molecules have been shown to block gene expression in a highly conserved regulatory mechanism known as RNA interference (RNAi).

However the in vivo application requires the nucleic acids specifically and efficiently interacting with target cells. The efficient in vivo delivery of functional therapeutic or diagnostic agents to a target tissue or cell still remains one of the biggest obstacles in drug development. Specific targeting and delivery of nucleic acids to and into target tissues and target cells is a major bottleneck, which has not satisfactorily been solved by current technologies. Most so far described nucleic acid delivery entities consist not of one defined molecule but rather are a cocktail of molecules or particles. However, for therapeutic applications, homogenous defined entities are desired.

The nucleic acid payloads must be attached covalently or non-covalently with good stability to assure specific targeting and avoid systemic nonspecific release of the payload. The carrier protects the nucleic acids from degradation in the extracellular environment. However, to enable entry into the cell, the payload is ideally released at or within target cells. To combine good stability within the circulation with effective release at the target is a major bottleneck in conjugate development. In addition the carrier has to be thus designed that it does not cause immunogenicity and is not toxic when accumulating in the body.

One approach is to attach the nucleic acids covalently or non-covalently to a polymeric carrier. For example polyamine structures have been widely used as artificial non-viral carrier systems. A major drawback of the polyamine carriers known so far is that they are random polymerization derived macromolecules and thus poorly defined, heterogeneous molecules in terms of their molecular weight and isomer distribution. The exact composition of these macromolecular polymeric systems is unknown and hence selective modification is impossible and structure activity relationships are difficult to predict.

Targeted nucleic acids frequently accumulate in endosomes from which they need to escape to be active. However, effective non-toxic, non-immunogenic endosome escape mechanisms for targeted dsRNAs still have to be found.

Hartmann et al. (e.g. Hartmann L, Hafele S, Peschka-Suss R, Antonietti M, Bomer HG. Tailor-Made Poly (amidoamine)s for Controlled Complexation and Condensation of DNA. Chemistry 2008;14(7):2025-2033.) describe linear PEG-polyamidoamines for controlled complexation and condensation of DNA, which are composed of propyleneamine subunits and sometimes combined with spermine. These structures are assembled using commercially avaliable PEG (2.7 kDa, ~63 repating units), diamino-N methyl-dipropylamine, spermine succinic acid anhydride, and lysine. Hartmann et al describe the successful synthesis optimization using a double condensation strategy and show successful complexation of DNA at high N/P ratios. However, these polymers are not suitable for successful siRNA/Oligo/pDNA delivery in vivo. None of the described structures contain lipophilic modifications or thiols for polyplex stabilization. Incorporation of a spacer peptide sequence is described but without any relevance to either polymer properties or delivery. Polymers were not optimized for pDNA binding or lytic activity. Polymer design is restricted by the boundaries of the synthesis and by the limited scope of commercial available amine building blocks. Furthermore the use of tri/tetramines results in a decreased charge density further weakening the nucleic acid complexation. Taken together all described sequences apparently possess no delivery potential. Substances were not tested for lytic activity but with regard to the published cytotoxicity results lytic activity is not very likely.

Wang et al (e.g. Wang XL, Ramusovic S, Nguyen T, Lu ZR, Novel polymerizable surfactants with pH-sensitive amphiphilicity and cell menrane disruption for efficient siRNA delivery, Bioconjugate Chemistry, 2007, 18, 2169-2177) specifically aimed at the development of amphiphilic cationic lipids with classical Y-shape. The substances are characterized by a maximum of 5 charges per molecule and a rather high HLB value. The synthetic route used does not allow the incorporation of additional amines. The structure is therefore not a polymer and could be described as a dipeptide and fatty acid modified oligoamine. Polymer design is restricted by the narrow boundaries of the synthesis and by the limited scope of commercial available amine building blocks. None of the described structures contain more than one amine building block and the synthesis is limited to commercially available amines. The synthesized structures are exclusively branched structures and have more similarity to PAMAM dendrons than to classical linear polyamidoamines as the amine building block is connected via an alkyl bond to the peptide domain. The polymer design is restricted by the boundaries of the synthesis and by the limited scope of commercial available amine building blocks. Furthermore the use of a single amine building block results in a decreased charge density compared to classical PAAs. Complex, multimodal, programmable delivery polymers can’t be synthesized by this strategy as its inherently restricted by its inability to incorporate more than one amino acid unit. Structures were tested for lytic activity and siRNA delivery but

show only moderate efficiency. DNA delivery results were not reported. Targeting/shielding can only be incorporated via post-pegylation approaches making the system less flexible and difficult to control.

These drawbacks of prior art are overcome by the polymers of the present invention, providing defined polymeric systems which can be used as effective carriers for the delivery of nucleic acids, with the ability to deliver the nucleic acids into the cytosol across cellular membranes, for example by delivery into and release from endosomal compartments. The polymers of the invention are pH specifically protonated and thus act as pH responsive lytic polymers.

Solid-phase synthesis of linear PEG-polyamidoamines was first reported by Hartmann et al. using a alternating condensation approach resulting in defined, linear, propylenimine based polyamidoamines with high purity. To generate a oligoethyleneimine based library the corresponding boc-protected oligoethylenimine building blocks were synthesized. Application of the published synthesis protocols on synthesis of the novel polymers comprising oligo(alkyleneamine) of this invention was not succesful as the reaction tended to generate crosslinked fragments on the resin. Therefore a new solid phase synthesis strategy based on fmoc/tBu was developed.

An object of the present invention is to provide a synthetically defined nucleic acid delivery carrier of low toxicity and high safety based on oligo(alkyleneamine) units. The carrier, when used to administer a nucleic acid such as a siRNA into an animal-derived cell or organism, is capable of delivering efficiently the nucleic acids into the cells while protecting it from being degraded. In one preferred embodiment, the carrier is (totally or partly) prepared by iterative solid-phase synthesis. The nucleic acid delivery formulation is prepared by either mixing the nucleic acid delivery carrier with a nucleic acid or covalently conjugating the nucleic acid to the carrier, or a combination of both. A polymer comprising oligo(alkyleneamino) acid units is provided, wherein the units have the general structure 1:

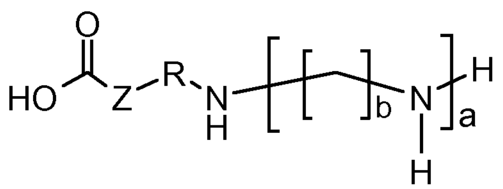

GENERAL STRUCTURE 1

wherein
*Z* is chosen from
either an alkyl group with the general structure

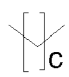

,

wherein the number *c* of methylene ($CH_2$) groups is 0-8;
an amino group with the general structure

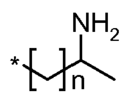

,

wherein the number *n* of methylene groups is 1-7;
or an aromatic group with the structure

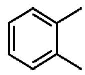, or 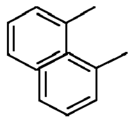 ;.

*R* is either a methylene group ($CH_2$) or a carbonylgroup (C=O); and
the number *a* of alkyleneamino ($(CH_2)_b$ NH) groups is 1-7, wherein the number *b* of methylene ($CH_2$) groups is 2-7 .

The oligo(alkyleneamino) acid units of the polymer are covalently linked via the terminal carboxygroup of a first oligo (alkyleneamino) acid unit and a terminal amino group of a second oligo(alkyleneamino) acid unit. Thus, the polymer comprises oligo (alkyleneamino) acid units covalently linked to each other via an amide bond, and is terminated by a free carboxyl group (-COOH) at the one end and terminated by a free amine group ($-NH_2$) at the other end. Hence the polymer has a C (carboxy) terminus and an N (amino) terminus.

Said polymer comprises 2-60 oligo(alkyleneamino) acid units having the general structure 1, preferably 2-10 oligo(alkyleneamino) acid units having the general structure 1, most preferably 2-5 oligo(alkyleneamino) acid units having the general structure 1. The polymer can optionally have the same oligo(alkyleneamino)acid units recurring or may also have a combination of varying oligo(alkyleneamino)acid units recurring.

In other embodiments said polymer comprises identical oligo(alkyleneamino)acid units and at least one amino acid recurring, in yet other embodiments said polymer comprises a combination of varying oligo(alkyleneamino)acid units and at least one amino acid recurring.

Below preferred embodiments of the oligo(alkyleneamino)acid units of general structure 1 are described.

As stated above, substituent Z of the oligo(alkyleneamino) acid units are selected from an alkyl group, an amino group or an aromatic group. Below preferred but not limiting embodiments are described wherein Z is an alkyl group.

In a preferred embodiment, *Z* is an alkyl group wherein the number *c* of methylene ($CH_2$) groups is 0-8; *R* is either a methylene group or a carbonylgroup; and the number *a* of alkyleneamino ($(CH_2)_b$ NH) groups is 1-7, wherein the number *b* of methylene ($CH_2$) groups is 2-7. Accordingly said oligo(alkyleneamino) acid units have the general structure 2:

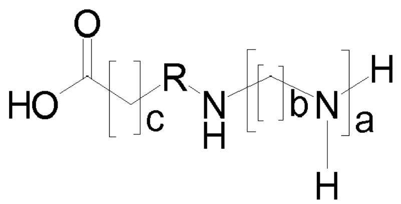

a: 2-7; b: 2-6, c: 0-8 GENERAL STRUCTURE 2

Said polymer comprises 2-60 oligo(alkyleneamino) acid units having the general structure 2, preferably 2-10 oligo(alkyleneamino) acid units having the general structure 2, most preferably 2-5 oligo(alkyleneamino) acid units having the general structure 2. The polymer can optionally have the same oligo(alkyleneamino)acid units recurring or may also have a combination of varying oligo(alkyleneamino)acid units recurring.

In a preferred embodiment, *Z* is an alkyl group wherein the number *c* of methylene ($CH_2$) groups is 0-8, *R* is a carbonylgroup, and the number *a* of alkyleneamino ($(CH_2)_b$ NH) groups is 1-7, wherein the number *b* of methylene ($CH_2$) groups is 2-7 . Accordingly said oligo(alkyleneamino) acid units have the general structure 3:

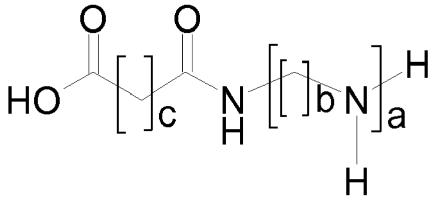

a: 2-7; b: 2-6, c: 0-8 GENERAL STRUCTURE 3

As illustrated above in the general structure 3, said oligo(alkyleneamino) acid units preferably comprise of 2 - 7, most preferably 3 or 4, alkyleneamine monomers wherein one alkyleneamine monomer is covalently linked to an aliphatic dicarboxylic acid. Preferably said alkyleneamine monomer is selected from the group: Ethyleneamine, propyleneamine, butyleneamine, pentylenamine and hexyleneamine. Preferably said dicarboxylic acid is an aliphatic dicarboxylic acid selected from the group oxalic acid, malonic acid, succinic acid, glutaric acid, adipic acid, pimelic acid, suberic acid, azelaic acid and sebacic acid. Said polymer comprises 2-60 oligo(alkyleneamino) acid units having the general structure 3, preferably 2-10 oligo(alkyleneamino) acid units having the general structure 3, most preferably 2-5 oligo (alkyleneamino) acid units having the general structure 3. The polymer can optionally have the same oligo(alkyleneamino) acid units recurring or may also have a combination of varying oligo(alkyleneamino)acid units recurring.

In a preferred embodiment, *Z* is an alkyl group wherein the number *c* of methylene ($CH_2$) groups is 0-8, *R* is a carbonylgroup, and the number *a* of alkyleneamino ($(CH_2)_b$ NH) groups is 1-7, wherein the number *b* of methylene ($CH_2$) groups is 2 . Accordingly in this embodiment said oligo(alkyleneamino) acid units are oligo (ethyleneamino) acid units, said units having the general structure 4:

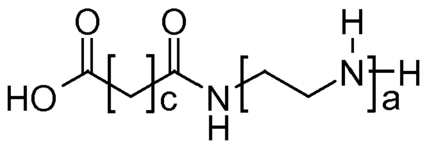

, wherein a: 2-7, c: 0-8.

GENERAL STRUCTURE 4

Said polymer comprises 2-60 oligo(ethyleneamino) acid units having the general structure 4, preferably 2-10 oligo (ethylenamino) acid units having the general structure 4, most preferably 2-5 oligo(ethylenamino) acid units having the general structure 4.

As illustrated above in the general structure 4, said oligo(ethyleneamino) acid units preferably comprise of 2 to 7 ethyleneamine monomers wherein one ethyleneamine monomer is covalently linked to a dicarboxylic acid. Preferably said dicarboxylic acid is an aliphatic dicarboxylic acid selected from the group oxalic acid, malonic acid, succinic acid, glutaric acid, adipic acid, pimelic acid, suberic acid, azelaic acid and sebacic acid.

In a preferred embodiment, *Z* is an alkyl group wherein the number *c* of methylene ($CH_2$) groups is 1, *R* is a carbonylgroup, and the number *a* of alkyleneamino ($(CH_2)_b$ NH) groups is 4, wherein the number *b* of methylene ($CH_2$) groups is 2. Hence the oligo(ethylenamino) acid units of this embodiment are tetraethylenepentamine succinic acid (Stp) units. Preferably the polymer comprises 2-5 of these (Stp) units.

In another embodiment, *Z* is an alkyl group wherein the number *c* of methylene ($CH_2$) groups is 0-8; *R* is a methylene group; and the number *a* of alkyleneamino ($(CH_2)_b$ NH) groups is 2-7, wherein the number *b* of methylene ($CH_2$) groups is 2-6. Accordingly said oligo(alkyleneamino) acid units have the general structure 5:

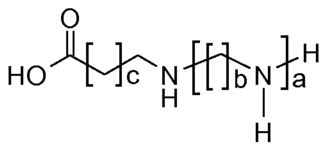

GENERAL STRUCTURE 5

a: 2-7; b: 2-6, c: 0-8

Said polymer comprises 2-60 oligo(alkyleneamino) acid units having the general structure 5, preferably 2-10 oligo(alkyleneamino) acid units having the general structure 5, most preferably 2-5 oligo(alkyleneamino) acid units having the general structure 5. The polymer can optionally have the same oligo(alkyleneamino)acid units recurring or may also have a combination of varying oligo(alkyleneamino)acid units recurring.

As illustrated above in the general structure 5, said oligo(alkyleneamino) acid units preferably comprise of 2 - 7 alkyleneamine monomers wherein one alkyleneamine monomer is covalently linked to a monocarboxylic acid. Preferably said alkyleneamine monomer is selected from the group: Ethyleneamine, propyleneamine, butyleneamine, pentylenamine and hexyleneamine. Preferably said carboxylic acid is an aliphatic carboxylic acid selected from the group acetic acid, propionic acid, butanoic acid, pentanoic acid, hexanoic acid, heptanoic acid, octanoic acid and nonaoic acid.

In another embodiment, *Z* is an alkyl group wherein the number *c* of methylene ($CH_2$) groups is 0-8; *R* is a methylene group; and the number *a* of alkyleneamino ($(CH_2)_b$ NH) groups is 2-7, wherein the number *b* of methylene ($CH_2$) groups is 2. Accordingly said oligo(alkyleneamino) acid units are oligo (ethyleneamino) acid units with the general structure 6

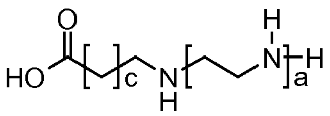

wherein a: 2-7, c: 0-8. GENERAL STRUCTURE 6

Said polymer comprises 2-60 oligo(ethyleneamino) acid units having the general structure 6, preferably 2-10 oligo(ethylenamino) acid units having the general structure 6, most preferably 2-5 oligo(ethylenamino) acid units having the general structure 6.

As illustrated above in the general structure 6, said oligo(ethyleneamino) acid units preferably comprise of 2 to 7 ethyleneamine monomers wherein one ethyleneamine monomer is covalently linked to a carboxylic acid. Preferably said carboxylic acid is an aliphatic carboxylic acid selected from the group acetic acid, propionic acid, butanoic acid, pentanoic acid, hexanoic acid, heptanoic acid, octanoic acid and nonaoic acid. As stated above, substituent Z of the oligo(alkyleneamino) acid units are selected from an alkyl group, an amino group or an aromatic group. Below preferred but not limiting embodiments are described wherein Z is an amino group of the general structure

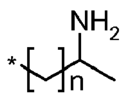

,

wherein the number *n* of methylene groups is 1-7. Preferably, R is a carbonyl group. In one preferred option, R is a carbonyl group and *n* is 1 or 2.

The oligo(alkyleneamino) acid polymers and derivatives described above are preferably synthesized by solid phase synthesis. The oligo(alkyleneamino) acid polymers provided herein are defined monodisperse molecules of low

toxicity with the ability to noncovalently bind nucleic acids. Nucleic acids bear anionic groups and can form an ionic bond with the polymers of this invention. The protonation of the alkyleneamine subunits of the polymers is pH dependent, therefore the payload is released specifically from the endosome. Hence in a preferred embodiment the oligo(alkyleneamino) acid polymers comprise a nucleic acid complexed or covalently conjugated to the polymer. Therefore the oligo (alkyleneamino) acid polymers of the invention are potent carrier molecules useful for the delivery of nucleic acids. In addition, the oligo(alkyleneamino) acid polymers can be designed according to the different requirements for the delivery of various nucleic acid species and are thus useful for the delivery of such different nucleic acid species like DNA, RNA, siRNA, LNA, PNA and others.

To meet the certain needs required for the successful in vivo delivery of nucleic acids, the oligo(alkyleneamino) acid polymers of the invention can be further modified. Targeted nucleic acids frequently accumulate in endosomes from which they need to escape to be active. It is known that the delivery of the nucleic acid into the cytoplasm can be facilitated by the addition of hydrophobic structures to the carrier molecule, as these hydrophobic structures increase the ability of the carrier to lyse lipid membranes. Due to the acidic environment of the endosomal/lysosomal compartments it is crucial for a nucleic acid delivery carrier to display a low buffering capacity at physiological pH values and a high buffering capacity at endosomal/lysosomal pH, thus facilitating selective endosomal/lysosomal membrane disruption. In one preferred embodiment of the invention the oligo(alkyleneamino) acid polymers additionally comprise a hydrophobic domain, which provides a pH-responsive lytic activity.

The hydrophobic domain comprises either multiple hydrophobic amino acid residues, such as leucine, valine, isoleucine, tyrosine or phenylalanine, or at least one fatty acid, such as, for example, myristic acid, palmitic acid, arachidic acid or other fatty acids. Preferred fatty acids include but are not limited to butyric acid, caprylic acid, myristic acid, oleic acid, linolic acid, arachidic acid, stearic acid, lauric acid and palmitic acid. Additionally, linker groups, between the hydrophobic domain and the oligo(alkyleneamino) acid polymer are also part of the invention. Preferably such linker groups are amino acids, most preferably lysine (for stable linkage) or cysteine (for bioreducible linkage). The use of naturally occurring amino acids or fatty acids is preferable to other hydrophobic moieties because they are relatively non-immunogenic and non-toxic. In addition, other hydrophobic domains such as hydrocarbon chains (saturated or unsaturated long chain aliphatic alcohols or thiols), stabilizing siRNA complexes and supporting endosome escape can be covalently linked to the polymers of the invention.

The hydrophobic domain described above is preferably covalently linked to the oligo(alkyleneamino) acid polymers of the invention.

In one embodiment, said hydrophobic domain is linked to the amine group at the N-terminus of the oligo (alkyleneamino) acid polymer, resulting in an i-shape as illustrated in figure 1 b.

In other embodiments, said hydrophobic domain is connected to the N- terminus of the oligo(alkyleneamino) acid polymer through a linker group. Said linker group is covalently linked to the amine group at the N-terminus of the polymer and comprises preferably at least one amino acid. Preferably said at least one amino acid is selected from the group of one lysine, two lysines covalently linked to each other via an amide bond, a cysteine and a lysine covalently linked to each other via an amide bond, and two cysteines covalently linked to each other via an amide bond.

In one embodiment the hydrophobic domain consists of two fatty acids covalently linked to a lysine residue via an amide bond using both amino functions. The lysine residue is either covalently connected to the N-terminus of the oligo(alkyleneamino) acid polymer via its carboxyl group, or covalently linked to the N-terminus of another aminoacid which is then connected to the N-terminus of the oligo(alkyleneamino) acid polymer via its carboxyl group. This second amino acid of the linker is preferably a lysine or a cysteine, as stated above. Said cysteine can be part of a crosslinking domain as stated below (see also Figure 1 d).

Other linker groups envisaged are amino acids with thiol groups such as cysteine. In this embodiment the hydrophobic domain is covalently linked to cysteine residues at the N-terminus of the oligo(alkyleneamino) acid polymer via a disulfide linkage.

In other embodiments said hydrophobic domain is covalently linked to two oligo(alkyleneamino) acid polymer chains of the invention, resulting in a t-shape as illustrated in figure 1 e. Preferably, said hydrophobic domain is connected to the oligo(alkyleneamino) acid polymers through a linker group. Said linker group is covalently linked to the two oligo (alkyleneamino) acid polymers via the amine group at the N-terminus or the carboxygroup at the C-terminus and comprises preferably at least one amino acid. Preferably said at least one amino acid is selected from the group of: one lysine, two lysines covalently linked to each other via an amide bond, a cysteine and a lysine covalently linked to each other via an amide bond, and two cysteines covalently linked to each other via an amide bond.

In one embodiment, said linker group is lysine, which is covalently linked to the amine group at the N-terminus of a first oligo(alkyleneamino) acid polymer chain and covalently linked to the carboxygroup of the C-terminus of a second oligo(alkyleneamino) acid polymer chain, and which is covalently linked via its second amine group with one fatty acid. In other embodiments, the second amine group is covalently linked to the carboxygroup of a lysine group, which is covalently linked to two fatty acids via an amide bond using both amino functions.

Apart from the t-shaped polymers described above, other branched oligo(alkyleneamino) acid polymers are

also part of this invention.

A key requirement for efficient transport using synthetic nucleic acid delivery systems is their ability to compact nucleic acids and the formation of stable polyplexes suitable for the efficient delivery to and into the cell. The nucleic acid compacting ability of polymers is strongly influenced by charge density, molecular weight and hydrophobic interactions of the polymers with the nucleic acid. Hence, structural modifications of the oligo(alkyleneamino) acid polymers that enhance compaction of the nucleic acid payload and formation of stable polyplexes are part of the invention. In one preferred embodiment, said polymer described above additionally comprises at least one head group which increases binding to the nucleic acid, formulation stability or interaction with lipid membranes.

The head group is preferably covalently linked to the N- or C-terminus of the oligo(alkyleneamino) acid polymers, preferably via a peptide bond. In one preferred embodiment the headgroup preferably comprises a polar positively charged head group, a coupling or crosslinking domain or a polar positively charged head group in the vicinity of a coupling or crosslinking domain.

Said coupling and crosslinking domain facilitates cross-linking of the oligo(alkyleneamino) acid polymers (dimerisation or polymerisation) and the coupling onto macromolecules such as nucleic acids. Thus the coupling or crosslinking domain increases self-stabilisation of oligo(alkyleneamino) acid polymers. The dimerisation/polymerisation of polymer molecules increases the positive charge and the number of aliphatic chains per molecule, which positively influence the transfection efficiency as well as the noncovalent binding of nucleic acids to the polymers. The resulting crosslinked polyplexes (i.e. crosslinked oligo(alkyleneamino) acid polymers) disintegrate after transport into the cell due to the bioreversible nature of the crosslinking. In addition, it is possible to covalently link the nucleic acid to the carrier polymer via the coupling and crosslinking domain.

Said coupling and crosslinking domain preferably comprises a functional group which facilitates bioreversible ligation, for example via disulfide bond formation, and comprises inter alia a cysteine, imine, hydrazone or azide. In one embodiment, the oligo(alkyleneamino) acid polymer comprises at least one, preferably two or three cysteine groups (Figure 1c to Figure 1f). Preferably the oligo(alkyleneamino) acid polymer comprises two cysteine groups covalently linked via a peptide bond to each terminus of the polymer. In one embodiment, the oligo(alkyleneamino) acid polymer additionally comprises a hydrophobic domain which is covalently linked to one of the terminal cysteine groups, which results in the i-shape topology (Figure 1 d). As stated above, the hydrophobic group is either covalently linked via a peptide bond to the cysteine group directly or via a lysine group that is covalently linked to the cysteine and the hydrophobic group. In another embodiment, a polymer molecule with t-shape conformation is envisaged, comprising two oligo(alkyleneamino) acid polymer chains to each of which a cysteine group is covalently linked to one terminus, said oligo(alkyleneamino) acid polymers being connected via a central linker group, and a lipophilic domain which is covalently linked to the central linker group (Figure 1 e). Preferably said linker group is at least one amino acid, most preferably one lysine or two lysines covalently linked to each other via a peptide bond.

In other embodiments, said head group comprises a polar head group. Said polar head group facilitates nucleic acid/formulation stability or lipid membrane interaction and preferably comprises a thiol group, such as cysteine and is preferably flanked by at least one polar positively charged amino acid, such as lysine, arginine or histidin. In one preferred embodiment, said oligo(alkyleneamino) acid polymer comprises a polar headgroup and a hydrophobic domain. In another preferred option, said oligo(alkyleneamino) acid polymer comprises a crosslinking domain and a hydrophobic domain. Said domains are covalently linked to the oligo(alkyleneamino) acid polymer, either directly via the amine group or via a linker group. Said domains can be thus linked to the oligo(alkyleneamino) acid polymer to form a chain, an i-shaped polymer or a t-shaped polymer. In addition, other configurations (branched polymers) are envisaged.

Further, in one embodiment the oligo(alkyleneamino) acid polymers additionally comprise agents that facilitate other functions in the eukaryotic cell, e.g. receptor recognition (targeting), internalization, release, nucleus localization and system stabilization. Such agent preferably comprises a targeting ligand, which recognizes tissue specific cell-surface structures, such as receptors. Most preferably said targeting ligand comprises a targeting peptide, a protein, a small molecule or a carbohydrate ligand.

In one preferred embodiment, said polymer described above additionally comprises a shielding moiety. Said shielding moiety is useful for in vivo stabilization of the oligo(alkyleneamino) acid polymer/ nucleic acid polyplex while circulating in the blood and to reduce undesiredinteractions or agglomeration oligo(alkyleneamino) acid polymer/ nucleic acid polyplex and preferably comprises monodisperse or polydisperse PEG.

In one preferred embodiment, said polymer comprises a targeting ligand attached to a monodisperse PEG which is attached to the polymer (Figure 1 g).

For shielding/stabilization purposes a PEG-chain (preferably monodisperse) of a Mw > 1 kDa can be attached at any position of the oligo(alkyleneamino) acid polymer using the epsilon-amino function of lysine or the amino function at the N-terminus.

In other embodiments, the oligo(alkyleneamino) acid polymers are combined with lipids, including also PEG-lipids. In antoher embodiment the oligo(alkyleneamino) acid polymers are combined with transfection reagents. Non-limiting examples of transfection reagents are lipoplexes, liposomes and cationic lipids.

In one preferred embodiment use of the oligo(alkyleneamino) acid polymers and its derivatives for the delivery of nucleic acids are envisaged. Particularly of interest herein is the delivery of siRNA into its target cell.

Another embodiment provides a pharmaceutical composition comprising the oligo(alkyleneamino) acid polymers and a nucleic acid, and may further comprise an agent that is complexed to the polymer. In particular, said pharmaceutical composistion comprises a oligo(alkyleneamino) acid polymers and an siRNA molecule. These pharmaceutical compositions are particularly useful in the inhibition of the expression of a target gene in a cell, a tissue or an organism. The pharmaceutical composition comprising the oligo(alkyleneamino) acid polymers of the invention may also comprise (a) pharmaceutically acceptable carrier(s), diluent(s) and/or excipient(s). Yet a further embodiment provides a method of treating a mammal, comprising administering the oligo(alkyleneamino) acid polymers complexed with a nucleic acid to a mammal.

Definitions:

The term "nucleic acid" as used herein means an oligomer or polymer composed of nucleotides, e.g., deoxyribonucleotides or ribonucleotides, or compounds produced synthetically (e.g., PNA as described in U.S. Pat. No. 5,948,902 and the references cited therein) which can hybridize with naturally occurring nucleic acids in a sequence specific manner analogous to that of two naturally occurring nucleic acids, e.g., can participate in Watson-Crick base pairing interactions. Non-naturally occurring nucleic acids are oligomers or polymers which contain nucleobase sequences which do not occur in nature, or species which contain functional equivalents of naturally occurring nucleobases, sugars, or inter-sugar linkages, like peptide nucleic acids (PNA), threose nucleic acids (TNA), locked nucleic acids (LNA), or glycerol nucleic acids (GNA). This term includes oligomers that contain the naturally occurring nucleic acid nucleobases adenine (A), guanine (G), thymine (T), cytosine (C) and uracil (U), as well as oligomers that contain base analogs or modified nucleobases. Nucleic acids can derive from a variety of natural sources such as viral, bacterial and eukaryotic DNAs and RNAs. Other nucleic acids can be derived from synthetic sources, and include any of the multiple oligonucleotides that are being manufactured for use as research reagents, diagnostic agents or potential and definite therapeutic agents. The term includes oligomers comprising of a single strand nucleic acid or a double strand nucleic acid.

The term "siRNA" as used herein refers to double-stranded RNA molecules (dsRNA) capable of blocking gene expression in a highly conserved regulatory mechanism known as RNA interference (RNAi). The term "double-stranded RNA", "dsRNA molecule", or "dsRNA", as used herein, refers to a ribonucleic acid molecule, or complex of ribonucleic acid molecules, having a duplex structure comprising two anti-parallel and substantially complementary nucleic acid strands. The dsRNAs may comprise naturally occurring nucleotides or at least one modified nucleotide, such as a 2'-O-methyl modified nucleotide, a nucleotide comprising a 5'-phosphorothioate group, and a terminal nucleotide linked to a cholesteryl derivative or dodecanoic acid bisdecylamide group. 2' modified nucleotides may have the additional advantage that certain immunostimulatory factors or cytokines are suppressed when the inventive dsRNA molecules are employed in vivo, for example in a medical setting. Alternatively and non-limiting, the modified nucleotide may be chosen from the group of: a 2'-deoxy-2'-fluoro modified nucleotide, a 2'-deoxy-modified nucleotide, a locked nucleotide, an abasic nucleotide, 2'-amino-modified nucleotide, 2'-alkyl-modified nucleotide, morpholino nucleotide, a phosphoramidate, and a non-natural base comprising nucleotide.The two strands forming the duplex structure may be different portions of one larger RNA molecule, or they may be separate RNA molecules. Where the two strands are part of one larger molecule, and therefore are connected by an uninterrupted chain of nucleotides between the 3'-end of one strand and the 5' end of the respective other strand forming the duplex structure, the connecting RNA chain is referred to as a "hairpin loop". The RNA strands may have the same or a different number of nucleotides. In addition to the duplex structure, a dsRNA may comprise one or more nucleotide overhangs. The nucleotides in said "overhangs" may comprise between 0 and 5 nucleotides, whereby "0" means no additional nucleotide(s) that form(s) an "overhang" and whereas "5" means five additional nucleotides on the individual strands of the dsRNA duplex. These optional "overhangs" are located in the 3' end of the individual strands. As will be detailed below, also dsRNA molecules which comprise only an "overhang" in one the two strands may be useful and even advantageous in context of this invention. The "overhang" comprises preferably between 0 and 2 nucleotides. Most preferably 2 "dT" (deoxythymidine) nucleotides are found at the 3' end of both strands of the dsRNA. Also 2 "U"(uracil) nucleotides can be used as overhangs at the 3' end of both strands of the dsRNA. Accordingly, a "nucleotide overhang" refers to the unpaired nucleotide or nucleotides that protrude from the duplex structure of a dsRNA when a 3'-end of one strand of the dsRNA extends beyond the 5'-end of the other strand, or vice versa. For example the antisense strand comprises 23 nucleotides and the sense strand comprises 21 nucleotides, forming a 2 nucleotide overhang at the 3' end of the antisense strand. Preferably, the 2 nucleotide overhang is fully complementary to the mRNA of the target gene. "Blunt" or "blunt end" means that there are no unpaired nucleotides at that end of the dsRNA, i.e., no nucleotide overhang. A "blunt ended" dsRNA is a dsRNA that is double-stranded over its entire length, i.e., no nucleotide overhang at either end of the molecule.

The term "antisense strand" refers to the strand of a dsRNA which includes a region that is substantially complementary to a target sequence. As used herein, the term "region of complementarity" refers to the region on the

antisense strand that is substantially complementary to a sequence, for example a target sequence. Where the region of complementarity is not fully complementary to the target sequence, the mismatches are most tolerated outside nucleotides 2-7 of the 5' terminus of the antisense strand

The term "sense strand," as used herein, refers to the strand of a dsRNA that includes a region that is substantially complementary to a region of the antisense strand. "Substantially complementary" means preferably at least 85% of the overlapping nucleotides in sense and antisense strand are complementary.

As used herein, the term "fatty acid" includes both saturated, i.e. an alkane chain as known in the art, having no double bonds between carbons of the chain and having the maximum number of hydrogen atoms, and unsaturated, i.e. an alkene or alkyne chain, having at least one double or alternatively triple bond between carbons of the chain, respectively, and further terminating the chain in a carboxylic acid as is commonly known in the art, wherein the hydrocarbon chain is not less then four carbon atoms.

The process of delivering a nucleic acid to a cell has been commonly termed transfection or the process of transfecting. The term transfecting as used herein refers to the introduction of a nucleic acid or other biologically active compound from outside a cell to inside cell such the nucleic acid has biologically activity. The nucleic acid may be used for research purposes or to produce a change in a cell that can be therapeutic. The delivery of a nucleic acid can lead to modification of the genetic material present in the target cell. A transfection reagent or delivery vehicle is a compound or compounds that bind(s) to or complex(es) with oligonucleotides and nucleic acids, and mediates their entry into cells.

*In vitro* transfection reagents, or delivery vehicles, are compounds or compositions of compounds that bind to or complex with nucleic acids and mediate their entry into cells. Examples of transfection reagents include, but are not limited to, protein and polymer complexes (polyplexes), lipids and liposomes (lipoplexes), combinations of polymers and lipids (lipopolyplexes), calcium phosphate precipitates, and dendrimers. Typically, the transfection reagent has a component with a net positive charge that binds to the oligonucleotide's or nucleic acid's negative charge. Cationic transfection agents may also condense large nucleic acids. Transfection agents may also be used to associate functional groups with a nucleic acid. Functional groups include cell targeting signals, nuclear localization signals, compounds that enhance release of contents from endosomes or other intracellular vesicles (such as membrane active compounds), and other compounds that alter the behavior or interactions of the compound or complex to which they are attached (interaction modifiers).

As used herein, a "pharmaceutical composition" comprises a pharmacologically effective amount of a nucleic acid complexed with the oligo(alkyleneamino) acid polymers of the invention and a pharmaceutically acceptable carrier. However, such a "pharmaceutical composition" may also comprise individual strands of such a nucleic acid molecule. As used herein, "pharmacologically effective amount," "therapeutically effective amount" or simply "effective amount" refers to that amount of an nucleic acid effective to produce the intended pharmacological, therapeutic or preventive result.

The term "pharmaceutically acceptable carrier" refers to a carrier for administration of a therapeutic agent. Such carriers include, but are not limited to, saline, buffered saline, dextrose, water, glycerol, ethanol, and combinations thereof. The term specifically excludes cell culture medium. For drugs administered orally, pharmaceutically acceptable carriers include, but are not limited to pharmaceutically acceptable excipients such as inert diluents, disintegrating agents, binding agents, lubricating agents, sweetening agents, flavoring agents, coloring agents and preservatives as known to persons skilled in the art.

It is in particular envisaged that the pharmaceutically acceptable carrier allows for the systemic administration of the nucleic acids complexed with the oligo(alkyleneamino) acid polymers of this invention. Whereas also the enteric administration is envisaged the parenteral administration and also transdermal or transmucosal (e.g. insufflation, buccal, vaginal, anal) administration as well was inhalation of the drug are feasible ways of administering to a patient in need of medical intervention the compounds of this invention. When parenteral administration is employed, this can comprise the direct injection of the compounds of this invention into the diseased tissue or at least in close proximity. However, also intravenous, intraarterial, subcutaneous, intramuscular, intraperitoneal, intradermal, intrathecal and other administrations of the compounds of this invention are within the skill of the artisan, for example the attending physician.

For intramuscular, subcutaneous and intravenous use, the pharmaceutical compositions of the invention will generally be provided in sterile aqueous solutions or suspensions, buffered to an appropriate pH and isotonicity. In a preferred embodiment, the carrier consists exclusively of an aqueous buffer. In this context, "exclusively" means no auxiliary agents or encapsulating substances are present which might affect or mediate uptake of nucleic acids in the cells. Aqueous suspensions according to the invention may include suspending agents such as cellulose derivatives, sodium alginate, polyvinyl-pyrrolidone and gum tragacanth, and a wetting agent such as lecithin. Suitable preservatives for aqueous suspensions include ethyl and n-propyl p-hydroxybenzoate.

The term "oligo(alkyleneamino) acid polymer" as used herein includes all variations and derivatives of polymers formed by oligo (alkylenamino) acid units of the general structure

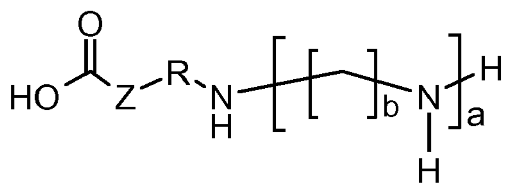

,

wherein
Z is chosen from either an alkyl group with the general structure

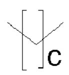

,

wherein *c* is 0-8; or an amino group with the general structure

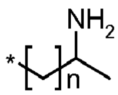

,

wherein *n* is 1-7; or an aromatic group with the structure

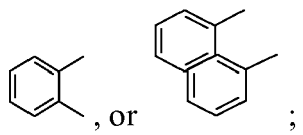

R is either $CH_2$ or C=O; and
*a* is 1-7 and *b* is 2-7.

The oligo(alkyleneamino) acid units of the polymer are covalently linked via the terminal carboxygroup of a first oligo (alkyleneamino) acid unit and a terminal aminegroup of a second oligo(alkyleneamino) acid unit. Thus, the polymer comprises oligo (alkyleneamino) acid units covalently linked to each other via an amide bond, and is terminated by a free carboxyl group (-COOH) at the one end and terminated by a free amine group ($-NH_2$) at the other end. Hence the polymer has a C (carboxy) terminus and an N (amino) terminus.

Said polymer comprises 2-60 oligo(alkyleneamino) acid units having the general structure 1, preferably 2-10 oligo(alkyleneamino) acid units having the general structure 1, most preferably 2-5 oligo(alkyleneamino) acid units having the general structure 1. The polymer can optionally have the same oligo(alkyleneamino)acid units recurring or may also have a combination of varying oligo(alkyleneamino)acid units recurring.

In other embodiments said polymer comprises identical oligo(alkyleneamino)acid units and at least one amino acid recurring, in yet other embodiments said polymer comprises a combination of varying oligo(alkyleneamino)acid units and at least one amino acid recurring.

A oligo(alkyleneamino) acid polymer can comprise more than one chain. Where the two oligo(alkyleneamino) acid polymer chains are connected covalently by means other than an uninterrupted chain of oligo(alkyleneamino) acid units, the connecting structure is referred to as a "linker". Mostly the linker is at least one amino acid. Through introduction of a linker into the oligo(alkyleneamino) acid polymer, branching of the oligo(alkyleneamino) acid polymer is possible. One example of a branched oligo(alkyleneamino) acid polymer is a t-shape, wherein two oligo(alkyleneamino) acid polymer chains are connected through an amino acid, to which a hydrophobic domain is covalently linked via another linker amino acid group. In addition to the t-shape, other branched oligo(alkyleneamino) acid polymers are envisaged with the oligo(alkyleneamino) acid polymer comprising 1-8 amino acids as branching points, preferably lysines and

cysteines. To these branching points within the oligo(alkyleneamino) acid polymer, more oligo(alkyleneamino) acid polymer chains are attached either on solid-phase or in solution, resulting in a multiple branched oligo(alkyleneamino) acid polymer.

If not stated otherwise all domains are connected by peptide bonds, linking the carboxy term of the domain covalently to the oligo(alkyleneamino) acid polymer. The peptide bond is the preferred option but can be substituted by bond types stable to the conditions of peptide synthesis either on solid-phase or in solution e.g. disulfide bond, Huisgen cycloaddition product: 1,2,3-triazole.

The term "head group" as used herein refers to any functional group covalently linked to the C or N-terminus of the oligo(alkyleneamino) acid polymer, preferably at the N-terminus, which increases binding to the nucleic acid, formulation stability or interaction with lipid membranes. The head group can either be a polar positively charged head group, a coupling or crosslinking domain or a polar positively charged head group in the vicinity of a coupling or crosslinking domain. Therefore the term "polar positively charged head group" refers to any polar positively charged functional group covalently linked to the C- or N-terminus of the oligo(alkyleneamino) acid polymer. Non-limiting examples of polar positively charged functional groups are thiol groups, such as cysteine, and polar positively charged amino acids like lysine, arginine or histidine. The term "coupling or crosslinking domain" refers to any functional group covalently linked to the C- or N-terminus of the oligo(alkyleneamino) acid polymer which enables crosslinking of the oligo(alkyleneamino) acid polymer (dimerisation or polymerisation). Said crosslinking is preferably bioreversible, therefore preferred functional groups of a crosslinking domain are disulfide-forming cysteine, oxime- or imine-forming aldehydes, or hydrazone-forming hydrazines.

The term "shielding moiety" as used herein refers to any moities that can be attached to the oligo(alkyleneamino) acid polymer resulting in a macromolecular structure to reduce unspecific interactions, uptake and agglomeration tendencies. For example a PEG-chain (preferably monodisperse) of a Mw > 1 kDa can be attached at any position of the polymersequence using the gamma-amino function of lysine or the N-terminus and provide effiecient shielding of the oligo(alkyleneamino) acid polymer.

The term "hydrophobic domain" as used herein refers to highly hydrophobic functional groups covalently attached of the oligo(alkyleneamino) acid polymer, preferably to the N-terminus. Highly hydrophobic groups include but are not limited to amino acids or fatty acids. Preferably two fatty acids or a stretch of hydrophobic amino acids are linked to the amino functions of a N-terminal lysine residue or the epsilon amino function of a branching lysine.

Short description of the figures:

**Figure 1 - Topological structures of the polymer** (a) chain b) - d) i-shape e) t-shape f) branched polymer g) shielded and targeted polymer.

**Figure 2 - Representative DNA gel-shift assay**. Comparison of the gel retardation of DNA of the different PAAs. All polymers were tested at a w/w of 10. Polymers showing no complete retardation at that concentration are shown in increasing concentrations. Picture 1 - 4 dimerizing i-shape family (67, 68, 69, 70); Picture 5 - 7 crosslinking i-shape family (45, 46, 51) 7 w/w 5,10,20; Picture 8 - 11 t-shape family (74, 49, 78, 82); 12 - 14 crosslinking chains family (72, 76, 80) w/w 5, 10

**Figure 3 - Representative RNA gel-shift assay**. Comparison of the gel retardation of siRNA of the different PAA families. Picture 1 - 4 dimerizing i-shape family (69, 70, 72, 71) N/P 12, 20; Picture 5 - 8 crosslinking i-shape family (50, 45, 46, 51) N/P 12, 20; Picture 9 -12 t-shape family (49, 76, 80, 84) N/P 6, 12

**Figure 4 - Dynamic light scattering**. Particle formation study of *in vivo* applied polymeric carrier systems. N/P ratio: molar ratio of protonable polymer nitrogens to DNA phosphates

**Figure 5 - Erythrocyte leakage assay**. Analysis of the lytic activity of the polymers at a concentration of 5 μM by erythrocyte leakage assay at different pHs.

**Figure 6 - Cell viability assay and Luciferase reporter gene expression**. Numbers in brackets depict ID numbers of polymers tested. (a) Reporter gene expression and metabolic activity of cells 24 h after transfection with pCMVLuc using i-shape PAA carrier systems. (b) Reporter gene expression and metabolic activity of cells 24h after transfection with pCMVLuc using t-shape PAA carrier systems (c) Reporter gene expression and metabolic activity of cells 24 h after transfection with pCMVLuc using non hydrophobical modified t-shape PAA carrier systems.

**Figure 7 - Cell viability assay and Luciferase reporter gene silencing**. (a) i- shapes. Comparison of different FA modifications (Polymer ID 45: C-Stp3-C-K-MyrA2, Polymer ID 46: C-Stp3-C-K- OleA2, Polymer ID 51: C-Stp3-C-K) - "Luc siRNA": SEQ ID No 1/2 , "Mut siRNA": SEQ ID No. 3/4 (b) t-shapes. Comparison one FA vs. 2FA

(Polymer ID 58: C-Stp2-K(O1eA)-Stp2-C, Polymer ID 49: C-Stp2-K-(K-OleA2)- Stp2-C) "Luc siRNA": SEQ ID No 1/2 , "Mut siRNA": SEQ ID No. 3/4 (c) Dimer-forming PAAs. (Polymer ID 72: C-Stp2-K- OleA2, Polymer ID 71: C-K-Stp2-K-01eA2), "Luc siRNA": SEQ ID No 1/2 , "Mut siRNA": SEQ ID No. 3/4 (d) TShape, Comparison on two different cell lines (Polymer ID 49: C-Stp2-K-(K-01eA2)-Stp2-C), "Luc siRNA": SEQ ID No 5/6 , "Mut siRNA": SEQ ID No. 7/8 (e) siRNA Knockdown comparison of two cross-linking i-shape homologues (Stp-backbone Polymer ID 46: C-Stp3-C-K-01eA2 vs. Gtp-backbone Polymer ID 213: C-$Gtp_3$-C-K-$OleA_2$). "Luc siRNA": SEQ ID No 5/6 , "Mut siRNA": SEQ ID No. 7/8.

**Figure 8 - Luciferase reporter gene expression of targeted polymers/cell viability (a) Reporter gene expression 24 h after transfection with targeted and shielded carrier systems**. HUH7 cells were transfected using 200 ng pCMVLuc (2 μg/mL DNA) plasmid. Polyplexes were prepared at different w/w ratios and compared to standard LPEI polyplexes. Luciferase reporter gene expression is presented as mean value + SD of quintuplicates. (b) **Metabolic activity of transfected cells 24 h after transfection with targeted and shielded carrier systems**. HUH7 cells were transfected using 200 ng pCMVLuc (2 μg/mL DNA) plasmid. Polyplexes were prepared at different w/w ratios and compared to standard LPEI polyplexes. Metabolic activity was measured by MTT assay and is presented as mean value + SD of quintuplicates.

**Figure 9 - In vivo luciferase reporter gene expression in different organs** (He, heart; Lu, lung; Li, liver; Sp, spleen; Ki, kidney; Tu, tumor; Mu, muscle; Va, isolated blood vessel) after systemic application. Systemic gene transfer in tumor bearing mice was carried out using polyplexes containing 50 μg pEGFPLuc DNA (Clontech Laboratories, Mountain View, CA) per 20 g body weight at a concentration of 200 μg/mL DNA in HBG and a polymer/DNA w/w ratio of 10. Results for polymers 46(Figure 9 a), 49(Figure 9 b), 82 (Figure c) are shown.

**Examples:**

Abbreviations

| | |
|---|---|
| C | cysteine |
| Bistfa | bis-trifluoroacetyl |
| Trisboc | tris-tert-butoxycarbonyl |
| Bisboc | bis-tert-butoxycarbonyl |
| Fmoc | (9-Fluorenylmethoxycarbonyloxy) |
| Fmoc-Stp(boc3)-OH | 1-(9-Fluorenylmethoxycarbonylamino)-3,6,9-tris(tert-butoxycarbonyl)-13-oxo-3,6,9,12-tetraazahexadecan-16-oic acid |
| RBF | Round-bottom flask |
| Fmoc-Osu | N-(9-Fluorenylmethoxycarbonyloxy) succinimide |
| DIPEA | Di-isopropylethyleneamine |
| Fmoc-Gtt-OH | 1-(9-Fluorenylmethoxycarbonylamino)-3,6,-bis(tert-butoxycarbonyl)-10-oxo-3,6,9-triazatetradecan-14-oic acid |
| Fmoc-Gtp-OH | 1-(9-Fluorenylmethoxycarbonylamino)-3,6,9-tris(tert-butoxycarbonyl)-13-oxo-3,6,9,12-tetraazaheptadecan-17-oic acid |
| TETA | Triethylenetetramine |
| TEPA | Tetraethylenepentamine |

| | |
|---|---|
| Pybop/HOBt | PyBOP®/1-Hydroxybenzotriazole |
| TFA | Trifluoroacetic acid |
| MTBE | Methyl tert-butyl ether |
| TFA/TIS | Trifluoroacetic acid/ Triisopropylsilane |
| Stp | Succinyltetraethylenepentamine (1-Amino-13-oxo-3,6,9,12-tetraazahexadecan-16-oic acid) |
| K | lysine |
| PAA | polyamide polymers |
| OAA | oligo (alkylene amino) acids |
| FA | fatty acid |

Synthesis of bistfa-trisboc-Tetraethylenepentamine (bistfa-trisboc-TEPA) using tetraethylenepentamine pentahydrochloride

10 g (26.9 mmol) tetraethylenepentamine pentahydrochloride were weighed in a 1 1 round bottom flask and dissolved in 250 ml dichloromethane (DCM) /methanol (2:1). 18.75 ml (134.5 mmol) triethylamine was added and after stirring for 2 - 12 h the mixture was cooled down to 0 °C. 6.72 ml (56.5 mmol) trifluoroacetic ethyl ester were diluted in dichloromethane (40 ml) and added dropwise over 2 h at 0 °C. The round bottom flask was allowed to warm to room temperature after 2 h and was stirred for another hour. Di-t-butyl dicarbonate (23.4 g, 107.6 mmol) was dissolved in 40 ml dichloromethane (DCM) and added dropwise over one hour. Afterwards 15 ml of triethylamine (107.5 mmol) were added and the mixture was stirred over night. The organic phase was reduced to approximately 150 ml and washed three times with saturated sodium bicarbonate, then three times with 5 % sodium citrate solution and finally three times with water. The organic phase was dried over sodium sulfate and the solvent was evaporated to a yellowish viscous, waxy solid. The residue was recrystallized: Therefore it was dissolved in the minimal amount of dichloromethane (37 ml) which was heated to reflux. Then slowly n-hexane (65 ml) was added to the boiling dichloromethane till clouding could be observed at the drop-in point. The crystallization solution was stored over night at 4 °C. The microcrystalline residue was filtered, washed with cooled n-hexane and dried, yielding 12.48 g (68.0 %) of bistfa-trisboc-tetraethylenepentamine.

1H-Nuclear Magnetic Resonance (NMR) spectrum (500 MHz, CDC13, 24.1 °C): $\delta$ = 1.39-1.48 (m, 27 H, OC (CH3)3), 3.20-3.55 (m, 16 H, CH2), 7.93 (d, J = 46.5,0.15 H, NH), 8.21 (d, J=41.3, 0.35H, NH) ppm.

Mass spectrometry analysis (MS), Electrospray injection (ESI); m/z (%) = 699.3527 [M+NH4]+ (100), 682.3268 [M+1]+ (22)

Synthesis of bistfa-trisboc-Tetraethylenepentamine using technical grade (85%) tetraethylenepentamine

12 g (53,8 mmol) of tetraethylenepentamine were weighed in a 1L round bottom flask and dissolved in 500 ml dichloromethane. The mixture was cooled down to 0 °C. Trifluoroacetic ethyl ester (13.45 ml, 16.05 g, 56.5 mmol) was diluted in 220 ml dichloromethane, transferred into a dropping funnel and added dropwise to the cooled mixture in the round bottom flask over 2.5 h. After complete addition of the trifluoroacetic ethyl ester the reaction was stirred for an additional hour at RT. Di-t-butyl dicarbonate (47 g, 215.3 mmol) was dissolved in 80 ml dichloromethane and added dropwise over one hour. Afterwards 30 ml (215 mmol) of triethylamine were added and the mixture was stirred over night. The organic phase was reduced to approximately 200 ml and washed three times with saturated sodium bicarbonate, then three times with 5 % sodium citrate solution and finally three times with water. The organic phase was dried over sodium sulfate and the solvent was evaporated to a yellowish viscous, waxy solid. The oily residue was recrystallised: Therefore it was solved in the minimal amount of boiling dichloromethane (60 ml). Then slowly n-hexane (140 ml) was added to the boiling dichloromethane till clouding was observed at the drop-in site. The crystallisation solution was stored over night in a refrigerator at 4 °C. The microcrystalline residue was filtered, washed with cooled n-hexane and dried.
Yield = 17.2 g (47%)
1H NMR (500 MHz, CDCl3, 24.1 °C): $\delta$ = 1.39-1.48 (m, 27 H, OC(CH3)3), 3.20-3.55 (m, 16 H, CH2), 7.93 (d, J = 46.5, 0.15 H, NH), 8.21 (d, J = 41.3, 0.35 H, NH) ppm.

MS (ESI); m/z (%) = 699.3527 [M+NH4]+ (100), 682.3268 [M+1]+ (22)

Synthesis of trisboc-Tetraethylenepentamine

10 g bistfa-trisboc-Tetraethylenepentamine were suspended in 75 ml ethanol. 100 ml of 3 M aqueous sodium hydroxide were slowly added via a dropping funnel under stirring. After a reaction time of 6-20 hours the alcohol was evaporated and the aqueous phase was extracted with 3 x 100 ml dichloromethane. The pooled organic phases were dried over sodium sulfate. After evaporation of the solvent and HV-treatment trisboc-tetraethylenepentamine was isolated in a 99.8 % yield as viscous oil which solidified slowly after addition of crystallization seeds and storage at 4 °C.
1H NMR (400 MHz, CDC13, 50.0 °C): δ = 1.40-1.50 (m, 27 H, OC(CH3)3), 2.36-2.58 (bs, 4 H, NH2), 2.79-2.96 (bt, J = 5.1, 4 H, CH2), 3.21 - 3.41 (m, 12 H, CH2) ppm.

MS (ESI); m/z (%) = 245.6837 [M+2H]+ (100), 490.3610 [M+H]+ (22)

Synthesis of bistfa-bisboc-triethylenetetramine (bistfa-bisboc-TETA)

Triethylenetetramine (TETA, 2.0 g, 13.7 mmol, 2.05 ml) was dissolved in 27 ml dichloromethane. A solution of trifluoroacetic ethyl ester (27) (4.09 g, 28.8 mmol, 3.43 ml) in 57 ml dichloromethane (99.5 %) was added dropwise at 0 °C and stirred for 1 h at 0°C. Triethylamine (2.91 g, 28.8 mmol, 4.00 ml,) was added and the reaction was brought to RT, followed by dropwise addition of a solution of di-t-butyl dicarbonate (8.9838 g, 41.2 mmol) in 21 ml dichloromethane. The reaction was stirred overnight and washed 3 x 5% NaHCO3 solution, 3 x 5% citric acid solution and 3 x water, dried over sodium sulfate, filtered and concentrated. The resulting white solid was recrystallized from dichloromethane/hexanes, yielding bistfa-bisboc-triethylenetetramine as a white solid.
Bistfa-bisboc-triethylenetetramine: 2.6745 g (36%).
1H NMR (500 MHz, CDCl3, 24.1 °C): δ = 1.40 (s, 9 H, OC(CH3)3), 1.46 (s, 9 H, OC(CH3)3), 3.24-3.63 (m, 12 H, CH2), 7.83 (sbr, 0.30 H, NH), 9.11 (sbr, 0.79 H, NH) ppm.

MS (ESI); m/z (%) = 556.2572 [M+NH4]+ (100), 539.2312 [M+1]+ (11)

Synthesis of bisboc-Triethylenetetramine

0.4882 g (0.907 mmol) of bistfa-bisboc-triethylenetetramine were suspended in 5 ml ethanol. While stirring 5 ml of aqueous 3 M NaOH were added dropwise over 10 min. After 16 h the solution was concentrated to approx. 5 ml and extracted with dichloromethane. The combined organic phase was dried over sodium sulfate and filtrated. The organic phase was evaporated yielding a crystalline solid which was further dried under high vacuum.
bisboc-Triethylenetetramine: 0.315 g (36%)
1H NMR (400 MHz, CDCl3, 19.1 °C): δ = 1.24 (sbr, 4 H, NH2), 1.45 (s, 18 H, OC(CH3)3), 2.812 (t, J = 6.2 Hz, 4 H, CH2), 3.25 (t, J = 6.2 Hz, 4 H, CH2), 3.32 (t, J = 7.5 Hz, 4 H, CH2) ppm.
13C NMR (125 MHz, CDCl3, 21.3 °C): δ = 28.8 (q, 6 C, C(CH3)3), 40.9 (t, 1 C, CH2), 41.3 (t, 1 C, CH2), 45.9 (t, 1 C, CH2), 46.2 (t, 1 C, CH2), 51.0 (t, 1 C, CH2), 51.6 (t, 1 C, CH2), 80.1 (q, 1 C, C(CH3)3), 80.2 (q, 1 C, C(CH3)3), 156.0 (s, 2 C, C=O) ppm.

Synthesis of Fmoc-Stp-OH

4.0 g of trisboc-tetraethylenepentamine (8.2 mmol) were dissolved in 16.5 ml of tetrahydrofuran and cooled to -75 °C. 0.514 g (0.5 mmol) of succinic anhydride were dissolved in 110 ml tetrahydrofuran and added dropwise over the course of 1 h. This was repeated once. The reaction was stirred for an additional hour at -75 °C and then for 1 h at RT. 4.19 ml hünig's base (DIPEA, mmol) were added to the RBF and the reaction mixture cooled to 0 °C. 4.128 g Fmoc-Osu (mmol) were dissolved in a mixture of acetonitril/ tetrahydrofuran (25 ml/ 12 ml). This solution was added dropwise to the reaction mixture and stirred over night. The solution was concentrated to approximately 100 ml, mixed with 100

ml of dichloromethane and was washed 5 x with 0.1 M sodium citrate buffer (pH 5.2). The organic phase was dried, concentrated and the resulting foamy, off-white product further purified by dry column vacuum chromatography using a n-heptane/ethyl acetate gradient to elute fmoc-byproducts, followed by a ethyl acetate /methanol gradient.
Fmoc-Stp-OH: 2,67 g (40%), bristle foamy, off-white solid.

1H-NMR (400 MHz, CDCl3, 19.1 °C): δ =

MS (ESI); m/z (%) =812.4419 [M+H]+ (42), 829.4682 [M+NH4]+ (100), 834.4237 [M+Na]+

Synthesis of Fmoc-Gtt-OH

Trisboc-TETA (8.65 g, 25 mmol) was dissolved in acetonitrile:tetrahydrofuran (200:50 ml) at room temperature and cooled to -70 °C, then dropwise glutaric anhydride solution (3.15 g, 27.6 mmol in 50 ml acetonitrile) was added for 20 min to obtain a solid mass. The reaction mixture was kept at same temp for 10 min then warmed up to room temperature and stirred for further 60 min. Hünig's base (8.34 g, 75 mmol) was added, the reaction mixture was cooled to 0 °C and stirred for further 15 min. Fmoc-OSu (12.60 g, 37.5 mmol in 250 ml acetonitrile) was added dropwise (1 h) and stirred for 20 min at the same temperature followed by warm up to room temperature and stirring it for 12 h. The solvent was removed under reduced pressure then dichloromethane (700 ml) was added and the organic phase was washed with sodium citrate hydrochloride solution (0.1 M, pH 5.5) five times, dried with sodium sulfate and evaporated to obtain a solid mass which was purified by dry column vacuum chromatography (Ethyl Acetate:Methanol; 9.5:0.5 to 7:3 v/v)
Yield: 7.35g, 42%
$^1$H NMR (400 MHz, $CDCl_3$): δ 7.75 (d, 2H, J=8Hz), 7.56 (d, 2H, J=8Hz), 7.38 (t, 2H, J=8Hz), 7.28 (t, 2H, J=8Hz), 4.40 (m, 2H), 4.20 (m, 1H), 3.32-3.41 (m, 12H), 2.37-2.40 (m, 2H), 2.26-2.29 (m, 2H), 1.91-1.98 (m, 2H), 1.45 (s, 27H).
$^{13}$C NMR (100 MHz, $CDCl_3$): δ 173 (C=O), 171 (C=O),143, 141, 128, 127, 125, 119 (Ar-C-Fmoc), 80 (CH-Fmoc), 60 (O$CH_2$-Fmoc), 47 ($CH_2$-TETA), 28 ($CH_3$-Tert-but), 21, 20 14 ($CH_2$-Glu)
High Resolution Mass Spectroscopy (HRMS), (EI): Calculated for $C_{36}H_{50}N_4O_9$, 682.3577, found: 681.3494, [M-1].

Synthesis of Fmoc-Gtp-OH

Trisboc-TEPA (9.8g, 20 mmol) was dissolved in tetrahydrofuran (250 ml) at room temperature and cooled to -70 °C, then glutaric anhydride solution (2.52 g, 24 mmol in 40 ml tetrahydrofuran) was added dropwise and the resulting suspension was stirred at -70 °C for 30 min, then brought to room temperature and stirred for further 60 min. Hünig's base (7.8 g, 60 mmol) was added and the solution was cooled to 0 °C. Fmoc-OSu (10.08 g, 30 mmol in 250 ml acetonitrile) was added dropwise (1 h), stirred for 20 min and was brought to room temperature, followed by stirring it for 12 h. The solvent was removed under reduced pressure followed by addition of dichloromethane (500 ml). The organic phase was washed with sodium citrate hydrochloride solution (0.1 M, pH 5.5) five times, dried with sodium sulfate and was evaporated to obtain a semi solid crude mass. The crude product was purified by dry column vacuum chromatography (ethyl acetate: methanol; 10:0 to 7:3 v/v)
Yield: 7.42g, 45%
$^1$H NMR (400 MHz, $CDCl_3$): δ 7.73 (d, 2H, J=8Hz), 7.55 (d, 2H, J=8Hz), 7.34 (t, 2H, J=8Hz), 7.26 (t, 2H, J=8Hz), 4.37 (m, 2H), 4.17 (m, 1H), 3.30-3.43 (m, 16H), 2.35-2.38 (m, 2H), 2.20-2.24 (m, 2H), 1.91-1.95 (m, 2H), 1.43 (s, 27H).
$^{13}$C NMR (100 MHz, $CDCl_3$): δ 172 (C=O, Glu), 171 (C=O, Glu),144, 141, 128, 127, 125, 119 (Ar-C-Fmoc), 80 (CH-Fmoc), 60 (O$CH_2$-Fmoc), 47, 45 ($CH_2$-TEPA), 35 ($CH_2$-Glu), 33 ($CH_2$-TEPA), 28 ($CH_3$-Ter-but), 21, 20 ($CH_2$-Glu), 14 ($CH_2$-Glu)
HRMS(EI): Calculated for $C_{43}H_{63}N_5O_{11}$, 825.4524, found: 824.4431, [M-1].

Solid-phase protocols

General Polyamidoamine synthesis protocol

| Reaction | Description | V [ml/g res] | Repititions/Time |
|---|---|---|---|
| 0a | DCM swell | 10 | 1 x 30 min |
| 1b | DMF wash | 10 | 3 x 1 min |
| 2 | 20% Pip/DMF prewash | 10 | 1 x 5 min |
| 3 | 20% Pip/DMF deprotection | 10 | 1 x 20 min |
| 4 | DMF wash | 10 | 5 x 1 min |
| 5 | Preactivated Fmoc-AA (4 eq) in DMF | 10 | 1 x 30 min |
| 6 | DMF wash | 10 | 5 x 1 min |
| 7c | Kaiser/TNBS test | | |

a Preswelling of trityl based resins before synthesis start

b Preswelling of wang based resins before synthesis start

c If Kaiser test does not show completion of coupling reaction (>99.5), repeat 4-7

Synthesis of Peptide precursors for targeting domain

Peptides were assembled in a fully automatic fashion using by fmoc/tBu chemistry on an Applied Biosystems 431A Peptide Synthesizer by employing the Applied Biosystems Small Scale FastMoc protocols.

General procedure: Manual synthesis of OAA-chains:

An amount of resin corresponding to 25-50 μmol of loaded amino acid was weighed into a syringe reactor and swelled for 30 min in an appropriate solvent. The general reaction cycle is depicted in the table above. Briefly, each

cycle began with fmoc-removal by treatment with 20% piperidine in DMF followed by DMF washing steps. Coupling was done using a mixture of protected OAA unit/Pybop/HOBt/DIPEA (4/4/4/8 equivalents (eq)) for 30 min or until complete conversion was indicated by Kaisertest.

General procedure: Synthesis of i-shapes terminated by one fatty acid (FA): exemplified by K-$Stp_1$-$FA_1$

After swelling of fmoc-K(boc)-Wang resin (0.05-0.20 mmol) in DMF and cleavage of the fmoc protecting group, 4 equivalents of a solution of fmoc-Stp-OH in DMF, 8 eq Hünigs base (DIPEA) and 4 eq PyBOP/HOBt were added to the resin and the vessel was agitated until Kaisertest indicated complete conversion (30 min). The reaction solvent was drained and the resin was washed five times with DMF. To cap residual, unreacted primary amino groups before introduction of the fatty acid the resin was acetylated using 5 equivalents of acetic anhydride and 10 equivalents of DIPEA in dichloromethane (DCM), before the subsequent removal of the fmoc protecting group. The resin was washed three times with DMF followed by three DCM washes after removal of the fmoc protecting group. Five equivalents of fatty acid were dissolved in DCM while 5 equivalents of PyBOP/HOBt and 10 equivalents of DIPEA dissolved in the smallest possible amount of DMF were added to the resin and the mixture was agitated until Kaisertest indicated complete conversion (normally 30 min). After completion of the reaction the resin was washed and dried for 12 h over KOH in vacuo. The PAA was cleaved from the resin by suspending it in a solution of TFA/H2O (95:5, v/v) for 3 h. The cleavage solution was collected by filtration and the resin washed twice with TFA and once with DCM. The collected solutions were concentrated under reduced pressure. The residue was dissolved in 5 % acetic acid and lyophilised.

General Procedure: Synthesis of i-shapes terminated by 2 fatty acids: exemplified by K-$Stp_1$-K- $FA_2$

After swelling of fmoc-Lys(boc)-Wang resin (0.05-0.20 mmol) in DMF and cleavage of the fmoc protecting group, four equivalents of a solution of fmoc-Stp-OH in DMF, DIPEA (8 eq) and PyBOP/HOBt (4 eq) were added to the resin and the vessel was agitated until Kaisertest indicated complete conversion (30 min). The reaction solvent was drained and the resin was washed five times with DMF. To covalently attach two fatty acids to the N-terminus of the PAA, fmoc-Lys(fmoc)-OH was incorporated before the coupling of the fatty acid. To cap residual, unreacted primary amino groups before introduction of the fatty acid the resin was acetylated using 5 equivalents of acetic anhydride and 10 equivalents of DIPEA in DMF, before subsequent removal of the fmoc protecting group. The resin was washed three times with DMF followed by three DCM washes after removal of the fmoc protecting group. Ten equivalents of the fatty acid were dissolved in DCM while 10 equivalents of PyBOP/ HOBt and 20 equivalents of DIPEA in the smallest possible amount of DMF were added to the resin and the mixture was agitated until Kaisertest did indicate complete conversion (normally 30 min). After completion of the reaction the resin was washed and dried for 12 h over KOH in vacuo. The PAA was cleaved from the resin by suspending it in a solution of TFA/H2O (95:5, v/v) for 3 h. The cleavage solution was collected by filtration and the resin washed twice with TFA and once with DCM. The collected solutions were concentrated under reduced pressure. The residue was dissolved in 5 % acetic acid and lyophilised.

General Procedure: Synthesis of i-shapes with a single coupling domain: exemplified by C-$Stp_1$-K- $FA_2$

For PAAs containing a C-terminal cysteine fmoc-Cys(trt)-Wang resin was used. Other steps of the synthesis were performed as described in General Procedure: synthesis of i-shapes terminated by 2 fatty acids. For cleavage the resin was suspended in a solution of TFA/TIS/H2O (95:2.5:2.5, v/v) and agitated for 3 h. The cleavage solution was drained and collected. The resin was washed twice with TFA and twice with DCM. The collected solutions were concentrated under reduced pressure to approximately 1 ml. The concentrated solution was dropped slowly in a 1:1 mixture (40 ml) of cooled (0 °C) MTBE and n-hexane. The resulting precipitate was centrifuged at 4 °C for 10 min (3000 rpm). The solvent was decanted and the pellet was washed twice with ice-cold MTBE. The resulting pellet was dissolved in 5 % acetic acid and lyophilised.

General Procedure: Synthesis of i-shapes with two coupling domains: exemplified by C-$Stp_3$-C- K- $FA_2$

After swelling 0.035 mmol of a fmoc-Cys(trt)-Wang resin in DMF and cleavage of the fmoc protecting group, four equivalents of a solution of fmoc-Stp-fmoc in DMF, DIPEA (8 eq) and Pybop/HOBt (4 eq) were added to the resin and the vessel was agitated until Kaisertest indicated complete conversion (normally 30 min). The reaction solvent was drained and the resin was washed five times with DMF. This cycle was repeated twice. Afterwards the amino acid fmoc-Cys(trt)-OH was coupled. Then, in order to covalently attach two fatty acids to the linear PAA, fmoc-Lys(fmoc)-OH was incorporated N-terminal before the coupling of the fatty acid. To cap unreacted primary amino groups, the resin was acetylated using 5 equivalents of acetic anhydride and 10 equivalents of DIPEA in DMF, before the subsequent removal of the fmoc protecting group. To covalently attach the fatty acid, the solvent was changed to DCM after fmoc cleavage.

The resin was washed three times with DMF and DCM after removal of the fmoc protecting group. 10 equivalents of the fatty acid were dissolved in DCM, 20 equivalents of DIPEA and 20 equivalents of Pybop/ HOBt in DMF were added to the resin and the mixture was agitated for 30 min. After completion of the reaction the resin was washed and dried over KOH in vacuo. For cleavage the resin was suspended in a solution of TFA/TIS/H2O (95:2.5:2.5, v:v:v) and agitated for 3 h. The cleavage solution was drained and collected. The resin was washed twice with TFA and once with DCM. The collected solutions were concentrated under reduced pressure to approximately 1 ml. The concentrated solution was dropped slowly in a 1:1 mixture (40 ml) of cooled (0 °C) MTBE and n-hexane. The resulting precipitate was centrifuged at 4 °C for 10 min (2000-3000 rpm). The solvents were decanted and the pellet was washed twice with ice-cold MTBE. The resulting pellet was dissolved in 5 % acetic acid and lyophilised.

General Procedure: Synthesis of T-shapes with one FA: exemplified by C-$Stp_1$-K (FA)-$Stp_1$-C

After swelling 0.05-0.20 mmol of fmoc-Cys(trt)-Wang resin in DMF for 30 min the fmoc-protection group was cleaved by double treatment with 20% piperidine in DMF. After washing the resin, four equivalents (related to resin loading) of fmoc-Stp-OH, DIPEA (8 eq) and Pybop/ HOBt (4 eq) were added for 30 min. The reaction solvent was drained and the resin was washed five times with DMF. Reaction progress was monitored by Kaisertest. To introduce a branching point dde-Lys(fmoc)-OH was used in the next coupling step. Dde-Lys(fmoc)-OH (4 eq) solved in DMF, DIPEA (8 eq) and Pybop/ HOBt (4 eq) solved in DMF were added and the synthesis vessel was agitated for 30 min. After a negative Kaiser test, the resin was washed with DMF. To cap unreacted primary amino groups, the resin was acetylated using 5 equivalents of acetic anhydride and 10 equivalents of DIPEA in DMF, before the subsequent removal of the fmoc protecting group. To couple the fatty acid, the solvent was changed to DCM after fmoc-cleavage. Therefore the resin was washed three times with DMF and DCM after removal of the fmoc-protecting group. 5 equivalents of the fatty acid solved in DCM, 10 equivalents of DIPEA and 5 equivalents of Pybop/ HOBt were added to the resin for 30 min. After completion of the reaction the resin was washed five times with DCM and three times with DMF. The dde-protecting group was cleaved with 2 % hydrazine monohydrate in DMF (v/v) (5-10 times for 5 min) till no significant $A_{300}$ was measurable in the deprotection mixture. In-between the deprotection-steps the resin was washed twice with DMF. Fmoc-Stp-OH dissolved in DMF, DIPEA (8 eq) and Pybop/ HOBt (4 eq) were added for 30 min. After a successful reaction the resin was treated twice with 20 % piperidine in DMF. After washing the resin, boc-Cys(trt)-OH (4 eq) solved in DMF, DIPEA (8 eq) and Pybop/ HOBt (4 eq) were added and the vessel agitated for 30 min. Afterwards the resin was washed and dried over KOH in vacuo. For cleavage the resin was suspended in a solution of TFA/TIS/H2O (95:2.5:2.5, v/v/v) and agitated for 3 h. The cleavage solution was drained and collected. The resin was washed twice with TFA and once with DCM. The collected solutions were concentrated under reduced pressure to approximately 1 ml. The concentrated solution was dropped slowly in a 1:1 mixture (40 ml) of cooled (0 °C) MTBE and n-hexane. The resulting precipitate was centrifuged at 4 °C for 10 min (2000-3000 rpm). The solvents were decanted and the pellet was washed twice with ice-cold MTBE. The resulting pellet was dissolved in 5 % acetic acid and lyophilised.

General Procedure: Synthesis of T-shapes with two FAs: exemplified by C-$Stp_1$-K (K- $FA_2$ -$Stp_1$-C

After swelling 0.05-0.20 mmol of fmoc-Cys(trt)-Wang resin in DMF for 30 min the fmoc-protection group was cleaved by double treatment with 20 % piperidine in DMF. After washing the resin, four equivalents (related to resin loading) of fmoc-Stp-OH, DIPEA (8 eq) and Pybop/ HOBt (4 eq) were added for 30 min. The reaction solvent was drained and the resin was washed five times with DMF. Reaction progress was monitored by Kaiser test. To introduce a branching point dde-Lys(fmoc)-OH was used in the next coupling step. Dde-Lys(fmoc)-OH (4 eq) solved in DMF, DIPEA (8 eq) and Pybop/ HOBt (4 eq) solved in DMF were added and the synthesis vessel was agitated for 30 min. After a negative Kaiser test, the resin was washed with DMF. After treatment with 20% piperidine in DMF and washing the resin with DMF, fmoc-Lys(fmoc)-OH (4 eq), DIPEA (8 eq) and Pybop/ HOBt (4 eq) was added. In order to cap unreacted primary amino groups, the resin was acetylated using 5 equivalents of acetic anhydride and 10 equivalents of DIPEA, before the subsequent removal of the fmoc protecting group. To couple the fatty acid, the solvent was changed to DCM after fmoc-cleavage. Therefore the resin was washed three times with DMF and DCM after removal of the fmoc-protecting group. 10 equivalents of the fatty acid solved in DCM, 20 equivalents of DIPEA and 10 equivalents of Pybop/ HOBt were added to the resin for 30 min. After completion of the reaction the resin was washed five times with DCM and three times with DMF. The dde-protecting group was cleaved with 2 % hydrazine monohydrate in DMF (v/v) (5-10 times for 5 min) till no significant $A_{300}$ was measurable in the deprotection mixture. In-between the deprotection-steps the resin was washed twice with DMF. fmoc-Stp-OH solved in DMF, DIPEA (8 eq) and Pybop/ HOBt (4 eq) were added for 30 min. After successful reaction the resin was treated twice with 20 % piperidine in DMF. After washing the resin, boc-Cys (trt)-OH (4 eq) solved in DMF, DIPEA (8 eq) and Pybop/ HOBt (4 eq) were added for 30 min. Afterwards the resin was washed and dried over KOH in vacuo. For cleavage the resin was suspended in a solution of TFA/TIS/H2O (95/2.5/2.5, v/v/v) and agitated for 3 h. The cleavage solution was drained and collected. The resin was washed twice with TFA and

once with DCM. The collected solutions were concentrated under reduced pressure to approximately 1 ml. The concentrated solution was dropped slowly in a 1:1 mixture (40 ml) of cooled (0 °C) MTBE and n-hexane. The resulting precipitate was centrifuged at 4 °C for 10 min (2000-3000 rpm). The solvents were decanted and the pellet was washed twice with ice-cold MTBE. The resulting pellet was dissolved in 5 % acetic acid and lyophilised.

General Procedure: Synthesis of T-Shapes containing shielding and targeting domains: exemplified by HO-IVNQPTY-GYWHY -$PEG_{24}$-K-($Stp_4$-C)$_2$:

After swelling 20 μmol of resin-bound HO-IVNQPTYGYWHY-$NH_2$ (GE11) in DMF for 30 min four equivalents of a solution of fmoc-$PEG_{24}$-OH in DCM/DMF(1:1, v/v), DIPEA (8 eq) and PyBOP/HOBt (4 eq) were added to the resin and the vessel was agitated until Kaisertest indicated complete conversion (60 min). The reaction solvent was drained and the resin was washed three times with DCM followed by three DMF washs. After removal of the fmoc protective group by treatment with 20% piperidine in DMF fmoc-Lys(fmoc)-OH was introduced as a branching point using standard coupling conditions (AA/PyBOP/HOBt/DIPEA, 4/4/4/8 eq in DMF). After succesfull deprotection 8 equivalents of fmoc-Stp-OH, 8 equivalents of PyBOP/HOBt and 16 equivalents of DIPEA in the smallest possible amount of DMF were added to the resin and the mixture was agitated until Kaisertest did indicate complete conversion (normally 30 min). The fmoc group was removed and the whole cycle repeated 4 times exchanging Stp by fmoc-C(Trt)-OH in the last step. After completion of the reaction the resin was washed and dried for 12 h over KOH in vacuo. The PAA was cleaved from the resin by suspending it in a solution of TFA/$H_2O$/TIS/EDT (92.5:2.5:2.5:2.5, v/v) for 1 h. The cleavage solution was collected by filtration and the resin washed twice with TFA and once with DCM. The concentrated solution was dropped slowly in a 1:1 mixture (40 ml) of cooled (0 °C) MTBE and n-hexane. The resulting precipitate was centrifuged at 4 °C for 10 min (2000-3000 rpm). The solvents were decanted and the pellet was washed twice with ice-cold MTBE. The resulting pellet was dissolved in 5 % acetic acid and lyophilised.

General cleavage procedure:

The resin was transferred into a syringe reactor of appropriate size and treated with 10 ml/g(resin) of a TFA/Water/TIS (95:2.5:2.5) mixture for 3 h. The resin was filtered off and washed twice using pure TFA followed by two DCM washes. The combined filtrates were concentrated in a rotovap and either precipitated by dropwise addition into ice-cold MTBE (50 ml MTBE/1 ml TFA) or other suitable mixtures. If precipitation wasn't possible the TFA was further concentrated to a glassy film and washed 3x with ice-cold MTBE. The precipitate/film was dissolved in 5% acetic acid, snap-frozen and lyophilized to obtain the crude peptide. Above described are general methods which can be applied for the synthesis of many different variations of oligo(alkyleneamino) acid polymers comprising oligo(alkyleneamino) acid subunits of the general structure 1. Non-limiting examples of oligo(alkyleneamino) acid polymer derivatives are listed in the table below:

**Table 1: Examples of Oligo(alkyleneamino) acid polymer derivatives**. Abbreviations: K= Lysine, C= Cysteine, Stp = Succinyl tetraethylenepentamine , OleA= Oleic acid, MyrA=Myristic Acid, CapA=Caprylic acid, ButA= Butyric acid, AraA= Arachidic acid, SteA= Stearic acid, prot a = protonable amines per molecule.

| Pol ID | Mol Weight | Formula | Sequence | prot a. | Type | C for coupling |
|---|---|---|---|---|---|---|
| 41 | 681.99 | $C_{36}H_{71}N_7O_5$ | K-$Stp_1$-$OleA_1$ | 4 | Chain | no |
| 40 | 627.90 | $C_{32}H_{65}N_7O_5$ | K-$Stp_1$-$MyrA_1$ | 4 | Chain | no |
| 39 | 543.74 | $C_{26}H_{53}N_7O_5$ | K-$Stp_1$-$CapA_1$ | 4 | Chain | no |
| 38 | 487.64 | $C_{22}H_{45}N_7O_5$ | K-$Stp_1$-$ButA_1$ | 4 | Chain | no |
| 37 | 953.35 | $C_{48}H_{96}N_{12}O_7$ | K-$Stp_2$-OleA | 7 | Chain | no |
| 36 | 899.26 | $C_{44}H_{9}0N_{12}O_7$ | K-$Stp_2$-$MyrA_1$ | 7 | Chain | no |
| 35 | 815.10 | $C_{38}H_{78}N_{12}O_7$ | K-$Stp_2$-$CapA_1$ | 7 | Chain | no |
| 34 | 759.00 | $C_{34}H_{7}0N_{12}O_7$ | K-$Stp_2$-$ButA_1$ | 7 | Chain | no |
| 15 | 1617.33 | $C_{84}H_{165}N_{19}O_{11}$ | K-$Stp_3$-K-$OleA_2$ | 10 | Chain | no |

(continued)

| Pol ID | Mol Weight | Formula | Sequence | prot a. | Type | C for coupling |
|---|---|---|---|---|---|---|
| 14 | 1509.15 | $C_{76}H_{153}N_{19}O_{11}$ | K-$Stp_{3}$-K-$MyrA_{2}$ | 10 | Chain | no |
| 13 | 1340.83 | $C_{64}H_{129}N_{19}O_{11}$ | K-$Stp_{3}$-K-$CapA_{2}$ | 10 | Chain | no |
| 30 | 773.08 | $C_{37}H_{72}N_{8}O_{7}S$ | C-$Stp_{1}$-K-$CapA_{2}$ | 3 | Chain | yes |
| 29 | 941.40 | $C_{49}H_{96}N_{8}O_{7}S$ | C-$Stp_{1}$-K-$MyrA_{2}$ | 3 | Chain | yes |
| 33 | 1049.58 | $C_{57}H_{1}0_{8}N_{8}O_{7}S$ | C-$Stp_{1}$-K-$OleA_{2}$ | 3 | Chain | yes |
| 31 | 1053.61 | $C_{57}H_{112}N_{8}O_{7}S$ | C-$Stp_{1}$-K-$SteA_{2}$ | 3 | Chain | yes |
| 3 | 798.11 | $C_{4}0H_{79}N_{9}O_{7}$ | K-$Stp_{1}$-K-$CapA_{2}$ | 4 | Chain | no |
| 4 | 966.43 | $C_{52}H_{1}0_{3}N_{9}O_{7}$ | K-$Stp_{1}$-K-$MyrA_{2}$ | 4 | Chain | no |
| 5 | 1046.56 | $C_{58}H_{111}N_{9}O_{7}$ | K-$Stp_{1}$-K-$OleA_{2}$ | 4 | Chain | no |
| 1 | 545.72 | $C_{24}H_{51}N_{9}O_{5}$ | K-$Stp_{1}$-K | 6 | Chain | no |
| 8 | 1069.47 | $C_{52}H_{1}0_{4}N_{14}O_{9}$ | K-$Stp_{2}$-K-$CapA_{2}$ | 7 | Chain | no |
| 9 | 1237.79 | $C_{64}H_{128}N_{14}O_{9}$ | K-$Stp_{2}$-K-$MyrA_{2}$ | 7 | Chain | no |
| 10 | 1345.97 | $C_{72}H_{14}0N_{14}O_{9}$ | K-$Stp_{2}$-K-$OleA_{2}$ | 7 | Chain | no |
| 6 | 817.08 | $C_{36}H_{76}N_{14}O_{7}$ | K-$Stp_{2}$-K | 9 | Chain | no |
| 20 | 1612.19 | $C_{76}H_{154}N_{24}O_{13}$ | K-$Stp_{4}$-K-$CapA_{2}$ | 13 | Chain | no |
| 21 | 1780.51 | $C_{88}H_{178}N_{24}O_{13}$ | K-$Stp_{4}$-K-$MyrA_{2}$ | 13 | Chain | no |
| 22 | 1888.69 | $C_{96}H_{19}0N_{24}O_{13}$ | K-$Stp_{4}$-K-$OleA_{2}$ | 13 | Chain | no |
| 18 | 1359.80 | $C_{6}0H_{126}N_{24}O_{11}$ | K-$Stp_{4}$-K | 15 | Chain | no |
| 50 | 1948.83 | $C_{1}00H_{2}0_{2}N_{24}O_{13}$ | K-$Stp_{4}$-K-$ArA_{2}$ | 13 | Chain | no |
| 45 | 1587.26 | $C_{76}H_{151}N_{19}O_{12}S_{2}$ | C-$Stp_{3}$-C-K-$MyrA_{2}$ | 9 | I-shape | yes |
| 46 | 1695.44 | $C_{84}H_{163}N_{19}O_{12}S_{2}$ | C-$Stp_{3}$-C-K-$OleA_{2}$ | 9 | I-shape | yes |
| 51 | 1166.55 | $C_{48}H_{99}N_{19}O_{1}0S_{2}$ | C-$Stp_{3}$-C-K | 11 | I-shape | yes |
| 26 | 2051.87 | $C_{1}00H_{2}O_{3}N_{29}O_{15}$ | K-$Stp_{5}$-K-$MyrA_{2}$ | 16 | Chain | no |

(continued)

| Pol ID | Mol Weight | Formula | Sequence | prot a. | Type | C for coupling |
|---|---|---|---|---|---|---|
| 27 | 2160.05 | $C_{1}0_{8}H_{215}N_{29}O_{15}$ | K-St$_{P5}$-K-OleA$_{2}$ | 16 | Chain | no |
| 25 | 2220.19 | $C_{112}H_{227}N_{29}O_{15}$ | K-Stp$_{5}$-K-AraA$_{2}$ | 16 | Chain | no |
| 23 | 1631.16 | $C_{72}H_{151}N_{29}O_{13}$ | K-Stp$_{5}$-K | 18 | Chain | no |
| 56 | 1564.11 | $C_{68}H_{138}N_{24}O_{13}S_{2}$ | C-Stp$_{2}$-K (CapA)-Stp$_{2}$- | 13 | T-Shape | yes |
| 57 | 1648.27 | $C_{74}H_{15}0N_{24}O_{13}S_{2}$ | C-Stp$_{2}$-K (MyrA)-Stp$_{2}$- | 13 | T-Shape | yes |
| 58 | 1702.36 | $C_{78}H_{156}N_{24}O_{13}S_{2}$ | C-Stp$_{2}$-K (01eA)-Stp$_{2}$-C | 13 | T-Shape | yes |
| 59 | 1732.43 | $C_{8}0H_{162}N_{24}O_{13}S_{2}$ | C-Stp$_{2}$-K (ArA)-Stp$_{2}$-C | 13 | T-Shape | yes |
| 68 | 1818.47 | $C_{82}H_{164}N_{26}O_{15}S_{2}$ | C-Stp$_{2}$-K(K- | 13 | T-Shape | yes |
| 48 | 1986.79 | $C_{94}H_{188}N_{26}O_{15}S_{2}$ | C-Stp$_{2}$-K-(K-MyrA$_{2}$)- | 13 | T-Shape | yes |
| 49 | 2094.98 | $C_{1}0_{2}H_{2}00N_{26}O_{15}S_{2}$ | C-Stp$_{2}$-K-(K-OleA$_{2}$)- | 13 | T-Shape | yes |
| 64 | 2155.11 | $C_{1}0_{6}H_{212}N_{26}O_{15}S_{2}$ | C-Stp$_{2}$-K(K-ArA$_{2}$)- | 13 | T-Shape | yes |
| 69 | 1212.76 | $C_{61}H_{121}N_{13}O_{9}S$ | C-Stp$_{2}$-K-MyrA$_{2}$ | 6 | Chain | yes |
| 70 | 1340.93 | $C_{67}H_{133}N_{15}O_{1}0S$ | C-K-Stp$_{2}$-K-MyrA$_{2}$ | 7 | Chain | yes |
| 72 | 1320.94 | $C_{69}H_{133}N_{13}O_{9}S$ | C-Stp$_{2}$-K-OleicA$_{2}$ | 6 | Chain | yes |
| 71 | 1449.11 | $C_{75}H_{145}N_{15}O_{1}0S$ | C-K-Stp$_{2}$-K-OleA$_{2}$ | 7 | Chain | yes |
| 73 | 1444.07 | $C_{7}0H_{138}N_{16}O_{11}S_{2}$ | C-Stp$_{1}$-K(K-MyrA$_{2}$)- | 7 | T-shape | yes |
| 76 | 1552.26 | $C_{78}H_{15}0N_{16}O_{11}S_{2}$ | C-Stp$_{1}$-K(K-OleA$_{2}$)- | 7 | T-shape | yes |
| 75 | 1556.29 | $C_{78}H_{154}N_{16}O_{11}S_{2}$ | C-Stp$_{1}$-K(K-SteA$_{2}$)- | 7 | T-shape | yes |
| 74 | 1023.36 | $C_{42}H_{86}N_{16}O_{9}S_{2}$ | C-Stp$_{1}$-K (K)-Stp$_{1}$-C | 9 | T-shape | yes |
| 77 | 2529.51 | $C_{118}H_{238}N_{36}O_{19}S_{2}$ | C-Stp$_{3}$-K(K-MyrA$_{2}$)- | 19 | T-shape | yes |
| 80 | 2637.69 | $C_{126}H_{25}0N_{36}O_{19}S_{2}$ | C-St$_{P3}$-K(K-OleA$_{2}$)- | 17 | T-shape | yes |
| 79 | 2641.73 | $C_{126}H_{254}N_{36}O_{19}S_{2}$ | C-Stp$_{3}$-K(K-SteA$_{2}$)- | 17 | T-shape | yes |

(continued)

| Pol ID | Mol Weight | Formula | Sequence | prot a. | Type | C for coupling |
|---|---|---|---|---|---|---|
| 78 | 2108.80 | $C_{9}0H_{186}N_{36}O_{17}S_{2}$ | C-Stp$_3$-K (K)-Stp$_3$-C | 19 | T-shape | yes |
| 81 | 3074.21 | $C_{14}0H_{286}N_{48}O_{23}S_{2}$ | C-Stp$_4$-K(K-MyrA$_2$)- | 25 | T-shape | yes |
| 84 | 3182.39 | $C_{148}H_{298}N_{48}O_{23}S_{2}$ | C-Stp$_4$-K(K-OleA$_2$)- | 25 | T-shape | yes |
| 83 | 3186.42 | $C_{148}H_{3}0_{2}N_{48}O_{23}S_{2}$ | C-Stp$_4$-K(K-SteA$_2$)- | 25 | T-shape | yes |
| 82 | 2653.50 | $C_{112}H_{234}N_{48}O_{21}S_{2}$ | C-Stp$_4$-K (K)-Stp$_4$-C | 27 | T-shape | yes |
| 118 | 648.86 | $C_{27}H_{56}N_{1}0O_{6}S$ | C-K-Stp-K | 6 | chain | yes |
| 119 | 1069.57 | $C_{55}H_{1}0_{8}N_{1}0O_{8}S$ | C-K-Stp-K-MyrA$_2$ | 4 | chain | yes |
| 120 | 1177.75 | $C_{63}H_{12}0N_{1}0O_{8}S$ | C-K-Stp-K-OleA$_2$ | 4 | chain | yes |
| 115 | 648.86 | $C_{27}H_{56}N_{1}0O_{6}S$ | C-Stp-K-K | 6 | chain | yes |
| 116 | 1069.57 | $C_{55}H_{1}0_{8}N_{1}0O_{8}S$ | C-Stp-K-K-MyrA$_2$ | 4 | chain | yes |
| 117 | 1177.75 | $C_{63}H_{12}0N_{1}0O_{8}S$ | C-Stp-K-K-OleA$_2$ | 4 | chain | yes |
| 121 | 920.22 | $C_{39}H_{81}N_{15}O_{8}S$ | C-Stp$_2$-K-K | 9 | chain | yes |
| 122 | 1340.93 | $C_{67}H_{133}N_{15}O_{1}0S$ | C-Stp$_2$-K-K-MyrA$_2$ | 7 | chain | yes |
| 123 | 1449.11 | $C_{75}H_{145}N_{15}O_{1}0S$ | C-Stp$_2$-K-K-OleA$_2$ | 7 | chain | yes |
| 124 | 920.22 | $C_{39}H_{81}N_{15}O_{8}S$ | C-Stp-K-Stp-K | 9 | chain | yes |
| 125 | 1340.93 | $C_{67}H_{133}N_{15}O_{1}0S$ | C-Stp-K-Stp-K-MyrA$_2$ | 7 | chain | yes |
| 126 | 1449.11 | $C_{75}H_{145}N_{15}O_{1}0S$ | C-Stp-K-Stp-K-OleA$_2$ | 7 | chain | yes |
| 133 | 1191.58 | $C_{51}H_{1}0_{6}N_{2}0O_{1}0S$ | C-Stp$_3$-K-K | 12 | chain | yes |
| 134 | 1612.29 | $C_{79}H_{158}N_{2}0O_{12}S$ | C-Stp$_3$-K-K-MyrA$_2$ | 10 | chain | yes |
| 135 | 1720.47 | $C_{87}H_{17}0N_{2}0O_{12}S$ | C-Stp$_3$-K-K-OleA$_2$ | 10 | chain | yes |
| 136 | 1191.58 | $C_{51}H_{1}0_{6}N_{2}0O_{1}0S$ | C-Stp$_2$-K-Stp-K | 12 | chain | yes |
| 137 | 1612.29 | $C_{79}H_{158}N_{2}0O_{12}S$ | C-Stp$_2$-K-Stp-K-MyrA$_2$ | 10 | chain | yes |
| 138 | 1720.47 | $C_{87}H_{17}0N_{2}0O_{12}S$ | C-Stp$_2$-K-Stp-K-OleA$_2$ | 10 | chain | yes |

(continued)

| Pol ID | Mol Weight | Formula | Sequence | prot a. | Type | C for coupling |
|---|---|---|---|---|---|---|
| 139 | 1191.58 | $C_{51}H_{1}0_{6}N_{2}0O_{1}0S$ | C-Stp-K-$Stp_2$-K | 12 | chain | yes |
| 140 | 1612.29 | $C_{79}H_{158}N_{2}0O_{12}S$ | C-Stp-K-$Stp_2$-K-$MyrA_2$ | 10 | chain | yes |
| 141 | 1720.47 | $C_{87}H_{17}0N_{2}0O_{12}S$ | C-Stp-K-$Stp_2$-K-$OleA_2$ | 10 | chain | yes |
| 142 | 1191.58 | $C_{51}H_{1}0_{6}N_{2}0O_{1}0S$ | C-K-$Stp_3$-K | 12 | chain | yes |
| 143 | 1612.29 | $C_{79}H_{158}N_{2}0O_{12}S$ | C-K-$Stp_3$-K-$MyrA_2$ | 10 | chain | yes |
| 144 | 1720.47 | $C_{87}H_{17}ON_{2}0O_{12}S$ | C-K-$Stp_3$-K-$OleA_2$ | 10 | chain | yes |
| 11 | 1088.44 | C48H101N19O9 | K-Stp3-K | 12 | Chain | No |
| 212 | 1477.17 | $C_{77}H_{149}N_{15}O_{10}S$ | C-K-$Gtp_2$-K-$OleA_2$ | 7 | I-Shape | yes |
| 213 | 1737.52 | $C_{87}H_{169}N_{19}O_{12}S_2$ | C-$Gtp_3$-C-K-$OleA_2$ | 9 | I-Shape | yes |
| 214 | 1629.34 | $C_{79}H_{157}N_{19}O_{12}S_2$ | C-$Gtp_3$-C-K-$MyrA_2$ | 9 | I-Shape | yes |
| 215 | 2858.20 | $C_{154}H_{298}N_{30}O_{18}$ | K-(K-$Gtp_2$-K-$OleA_2)_2$ | 14 | Branched | no |
| 216 | 2641.84 | $C_{138}H_{274}N_{30}O_{18}$ | K-(K-$Gtp_2$-K-$MyrA_2)_2$ | 14 | Branched | no |
| 217 | 1391.03 | $C_{73}H_{139}N_{13}O_{10}S$ | C-K-$Gtt_2$-K-$OleA_2$ | 5 | I-Shape | yes |
| 218 | 1391.03 | $C_{73}H_{139}N_{13}O_{10}S$ | C-$Gtt_3$-C-K-$OleA_2$ | 6 | I-Shape | yes |
| 219 | 1680.43 | $C_{84}H_{162}N_{18}O_{12}S_2$ | C-Gtp-Gtt-Stp-C-K-$OleA_2$ | 8 | I-Shape | yes |
| 220 | 1637.36 | $C_{82}H_{157}N_{17}O_{12}S_2$ | C-Stp-Gtt-Gtt-C-K-$OleA_2$ | 8 | I-Shape | yes |
| 221 | 1694.45 | $C_{85}H_{164}N_{18}O_{12}S_2$ | C-$Gtp_2$-Gtt-C-K-$OleA_2$ | 8 | I-Shape | yes |
| 222 | 1666.40 | $C_{83}H_{160}N_{18}O_{12}S_2$ | C-Gtt-$Stp_2$-C-K-$OleA_2$ | 8 | I-Shape | yes |
| 223 | 1387.00 | $C_{73}H_{135}N_{13}O_{10}S$ | C-K-$Gtt_2$-K-$LinA_2$ | 5 | I-Shape | yes |
| 224 | 1445.08 | $C_{75}H_{141}N_{15}O_{10}S$ | C-K-$Stp_2$-K-$LinA_2$ | 7 | I-Shape | yes |
| 225 | 1473.14 | $C_{77}H_{145}N_{15}O_{10}S$ | C-K-$Gtp_2$-K-$LinA_2$ | 7 | I-Shape | yes |
| 226 | 1733.49 | $C_{87}H_{165}N_{19}O_{12}S_2$ | C-$Gtp_3$-C-K-$LinA_2$ | 9 | I-Shape | yes |

(continued)

| Pol ID | Mol Weight | Formula | Sequence | prot a. | Type | C for coupling |
|---|---|---|---|---|---|---|
| 228 | 1604.29 | $C_{81}H_{150}N_{16}O_{12}S_2$ | C-Gtt$_3$-C-K-LinA$_2$ | 6 | I-Shape | yes |
| 229 | 1691.41 | $C_{84}H_{159}N_{19}O_{12}S_2$ | C-Stp$_3$-C-K-LinA$_2$ | 9 | I-Shape | yes |
| 230 | 1676.40 | $C_{84}H_{158}N_{18}O_{12}S_2$ | C-Gtp-Gtt-Stp-C-K-LinA$_2$ | 8 | I-Shape | yes |
| 231 | 1633.33 | $C_{82}H_{153}N_{17}O_{12}S_2$ | C-Stp-Gtt$_2$-C-K-LinA$_2$ | 7 | I-Shape | yes |
| 232 | 1690.42 | $C_{85}H_{160}N_{18}O_{12}S_2$ | C-Gtp$_2$-Gtt-C-K-LinA$_2$ | 8 | I-Shape | yes |
| 233 | 1662.37 | $C_{83}H_{156}N_{18}O_{12}S_2$ | C-Gtt-Stp$_2$-C-K-LinA$_2$ | 8 | I-Shape | yes |
| 181 | 4425,37 | $C_{204}H_{360}N_{50}O_{57}$ | GE$_{11}$-PEG-K-(Stp$_3$)$_2$ | 20 | T-Shape | no |
| 182 | 4968,09 | $C_{228}H_{410}N_{60}$O61 | GE$_{11}$-PEG-K-(Stp$_4$)$_2$ | 26 | T-Shape | no |
| 185 | 4631,65 | $C_{210}H_{370}N_{52}O_{59}S_2$ | GE$_{11}$-PEG-K-(Stp$_3$-C)$_2$ | 20 | T-Shape | yes |
| 186 | 5174,37 | $C_{234}H_{420}N_{62}O_{63}S_2$ | GE$_{11}$-PEG-K-(Stp$_4$-C)$_2$ | 26 | T-Shape | yes |
| 189 | 5160,53 | $C_{246}H_{434}N_{52}O_{61}S_2$ | GE$_{11}$-PEG-K-(Stp$_3$-C-OleA)$_2$ | 18 | T-Shape | yes |
| 190 | 5703,25 | $C_{270}H_{484}N_{62}O_{65}S_2$ | GE$_{11}$-PEG-K-(Stp$_4$-C-OleA)$_2$ | 24 | T-Shape | yes |
| 195 | 5945,75 | $C_{294}H_{522}N_{56}O_{65}S_2$ | GE$_{11}$-PEG-K-(Stp$_3$-C-K-(OleA)$_2$)$_2$ | 18 | T-Shape | yes |
| 196 | 6488,47 | $C_{318}H_{572}N_{66}O_{69}S_2$ | GE$_{11}$-PEG-K-(Stp$_4$-C-K-(OleA)$_2$)$_2$ | 24 | T-Shape | yes |
| 183 | 2973,78 | $C_{132}H_{270}N_{35}O_{39}$ | A-PEG-K-(Stp$_3$)$_2$ | 20 | T-Shape | no |
| 184 | 3516,50 | $C_{156}H_{320}N_{45}O_{43}$ | A-PEG-K-(Stp$_4$)$_2$ | 26 | T-Shape | no |
| 187 | 3180,06 | $C_{138}H_{280}N_{37}O_{41}S_2$ | A-PEG-K-(Stp$_3$-C)$_2$ | 20 | T-Shape | yes |
| 188 | 3722,78 | $C_{162}H_{330}N_{47}O_{45}S_2$ | A-PEG-K-(Stp$_4$-C)$_2$ | 26 | T-Shape | yes |
| 191 | 3708,94 | $C_{174}H_{344}N_{37}O_{43}S_2$ | A-PEG-K-(Stp$_3$-C-OleA)$_2$ | 18 | T-Shape | yes |

(continued)

| Pol ID | Mol Weight | Formula | Sequence | prot a. | Type | C for coupling |
|---|---|---|---|---|---|---|
| 192 | 4251,66 | $C_{198}H_{394}N_{47}O_{47}S_2$ | A-PEG-K-(Stp$_4$-C-OleA)$_2$ | 24 | T-Shape | yes |
| 193 | 4494,16 | $C_{222}H_{432}N_{41}O_{47}S_2$ | A-PEG-K-(Stp$_3$-C-K-(oleA)$_2$)$_2$ | 18 | T-Shape | yes |
| 196 | 5036,88 | $C_{246}H_{482}N_{51}O_{51}S_2$ | A-PEG-K-(Stp$_4$-C-K-(oleA)$_2$)$_2$ | 24 | T-Shape | yes |

Calculation of the polymer/nucleic acid ratio for polyplex formation

Defined charge ratios of cationic polymer/anionic nucleic acid were tested. The charge ratio can be defined either as N/P ratio or as w/w ratio. The N/P ratio is the molar ratio of protonable polymer nitrogens to DNA phosphates. The w/w ratio is the polymer/DNA weight/weight ratio. The polymer/DNA weight/weight (w/w) ratios can be calculated from the corresponding N/P ratio by the formula: w/w ratio = (N/P ratio) x molecular weight of the polymer / (number of protonable polymer groups x molecular weight of nucleotide monomer). The average molecular weight of nucleotide monomers is 330 for DNA and 348 for RNA.

DNA polyplex formation

Polyplex formulations for DNA delivery were prepared as follows: 100 ng of DNA and the calculated amount of PAA were diluted in separate tubes in Hepes Buffered Glucose pH 7.4 (HBG). The polycation solution was added to the nucleic acid, rapidly mixed by pipetting up and down and incubated for 30-40 min at RT in order to form the polyplexes necessary for transfection and gel-shift experiments.

siRNA polyplex formation

Non-covalent polyplex formulations for siRNA delivery were prepared as follows: 500 ng of siRNA and the calculated amount of PAA were diluted in separate tubes in HBG. Then the polycation solution was added to the nucleic acid, mixed by pipetting and incubated for 30-40 min at RT in order to form the polyplexes necessary for transfection and gel-shift experiments.

DNA gel-shift assay

A 1% agarose gel was prepared by dissolving 1.2 g agarose (Sigma-Aldrich, Taufkirchen, Germany) in 120 ml TBE buffer and heating the mixture to 100 °C. After cooling down to approximately 50 °C, 120 μl Gel-Red were added and the gel was poured in the casting unit. Polyplex-samples containing 100 ng DNA, PAA, HBG-buffer and loading buffer were placed into the pockets after an incubation time of 30 min at RT. Electrophoresis was performed at 120 V for 80 min. Results of a representative DNA gel shift are shown in figure 2.

siRNA gel-shift assay

A 2.5% agarose gel was prepared by dissolving 3.0 g agarose (Sigma-Aldrich, Taufkirchen, Germany) in 120 ml TBE buffer and heating the mixture to 100 °C. After cooling down to approximately 50 °C, 120 μL Gel-Red () were added and the gel was poured in the casting unit. Polyplex-samples containing 500 ng siRNA, PAA, HBG-buffer and loading buffer were placed into the pockets after an incubation time of 30 min at RT. Electrophoresis was performed at 120 V for 40 min. Results of a representative RNA gel shift are shown in figure 3.

Erythrocyte leakage assay

Freshly collected, citrate buffered murine blood was washed by four centrifugation cycles, each in phosphate-buffered saline (PBS) at 2000 rpm (600-800 g) at 4 °C for 10 min. The erythrocytes in the pellet were counted. The pellet was then diluted with different PBS buffers (pH 7.4, 6.5 and 5.5) to 5 x 107 erythrocytes/ml. The suspension was always freshly prepared and used within 24 h. 75 μl of the PAA solutions prepared at different concentration and different pH-values were mixed with 75 μl erythrocyte suspension in a 96-well plate (NUNC, V-bottom, Denmark). After incubating the plates under constant shaking at 37 °C for 60 min, intact blood cells and cell debris was removed by centrifugation (4 °C, 600-800 g (2000 rpm), 10 min). 80 μl of the supernatant was transferred to a new 96-well plate (TPP 96F, Trasadingen, Switzerland). Haemoglobin absorption was determined at 405 nm using a microplate reader (Spectrafluor Plus, Tecan Autstria GmbH, Grödig, Austria). PBS-buffers with pH-values of 7.4, 6.5 and 5.5 were used as negative control, 1 % TritonX-100 in PBS as positive control. Haemolysis was defined as percent ((ODPAA - ODbuffer)/(ODTriton-X100 - ODbuffer))*100. Results are shown in figure 5.

Cell viability assay (MTT Assay)

The metabolic activity of the cells was determined using a methylthiazole tetrazolium (MTT) assay as follows: 10 μL per 100 μL of medium of a 5 mg/ml solution of MTT in sterile PBS-buffer was added to each well of the 96-well plate. After incubation for 1-2 h at 37 °C the medium was removed and the cells were frozen at -80 °C for at least 1 h. 200 μl DMSO were added and the samples were incubated under constant shaking at 37 °C for 30 min to dissolve the crystals completely. The optical absorbance was measured at 590 nm with a reference wavelength of 630 nm using a microplate reader (Spectrafluor Plus,Tecan Autstria GmbH, Grödig, Austria). The cell viability was defined as percent: ($OD_{pAA}$ treated cells/$OD_{Buffer}$ treated cells)*100. Results are shown in figures 6 - 8.

Luciferase reporter gene silencing

All experiments were performed in stably transfected Neuro2A-eGFPLuc cells. Cells were seeded in 96-well plates (TPP, Trasadingen, Switzerland) using 5000 cells per well 24 h prior to transfection. Transfection complexes containing siRNA were then added to cells in 100 μl culture medium containing 10% serum, 100 U/ml penicillin and 100 μg/ml streptomycin (final siRNA-concentration 367 nmol/l). 48 h after initial transfection medium was removed and cells were lysed in 50 μl 0.5X Promega cell lysis solution to measure the gene expression as described below. Transfections were performed in parallel using a specific target siRNA and a non-specific control siRNA to distinguish between specific gene silencing and unspecific knockdown of protein expression due to carrier toxicity. Qualitative information on the toxicity of the conjugates was obtained by diminution in luciferase expression upon delivery of the non-specific control siRNA compared to the luciferase expression from the same number of untreated control cells. Results are shown in figures 6-8. The used vector pCMVLuc has been described elsewhere (Plank C, Zatloukal K, Cotten M, Mechtler K, Wagner E. Bioconjug Chem. 1992, 3(6):533-9.).

Sequences of siRNAs employed in this study:

Luciferase-siRNA GL3 luciferase duplex:

5’-CUUACGCUGAGUACUUCGAdTdT-3’ (sense) [Seq ID. No 1]
5’-UCGAAGUACUCAGCGUAAGdTdT-3’ (antisense) [Seq ID. No 2]

control-siRNA (non-targeting control duplex):

5’-AUGUAUUGGCCUGUAUUAGUU-3’ (sense) [Seq ID. No 3]
5’-CUAAUACAGGCCAAUACAUUU-3’ (antisense) [Seq ID. No 4]

EGFPLuc-siRNA duplex:

5 ‘-AuAucAuGGccGAcAAGcAdTsdT-3 (sense) [Seq ID. No 5]
5 ‘-UGCUUGUCGGCcAUGAuAUdTsdT-3’ (antisense) [Seq ID. No 6]

Control siRNA duplex:

5’-AuGuAuuGGccuGuAuuAGdTsdT-3’ (sense) [Seq ID. No 7]
5 ‘-CuAAuAcAGGCcAAuAcAUdTsdT-3’(antisense) [Seq ID. No 8]

Luciferase reporter gene expression

Cells were plated in 96 well plates at a density of 10.000 cells per well 24 h prior to transfection. The polyplexes formed using 200 ng of pDNA/well were added to the cells in 100 μl culture medium containing 10% serum, 100 U/ml penicillin and 100 μg/ml streptomycin. LPEI polyplexes (Zou SM, Erbacher P, Remy JS, Behr JP. J Gene Med. 2000 Mar-Apr;2(2):128-34) formed in HBG using 200 ng of pDNA and 160 ng of linear polyethylenimine, (LPEI, molecular weight 22 kDa) /well were used for comparison. 24 h after initial transfection medium was removed and cells were lysed in 50 μl 0.5X Promega cell lysis solution to measure the gene expression. Luciferase activity was measured using a Lumat LB9507 instrument (Berthold, Bad Wildbad, Germany). Luciferase light units were recorded from an 20 μl aliquot of the cell lysate with 10 s integration time after automatic injection of freshly prepared luciferin using the luciferase assay system (Promega, Mannheim, Germany). Transfection efficiency was evaluated as relative light units (RLU) per number of seeded cells. Two ng of recombinant luciferase (Promega, Mannheim, Germany) corresponded to $10^7$ light units. Results are shown in figures 6-8.

Dynamic light scattering

Particle size of siRNA and DNA formulations was measured by laser-light scattering using a Zetasizer Nano ZS (Malvern Instruments, Worcestershire, U.K.). Therefore polyplexes were formed for 30-40 min at RT, containing 10 μg nucleic acid and the PAA at a molar ratio of PAA nitrogen to DNA phosphate of 6, 12 or 20 (N/P = 6, 12, 20). The polyplexes were diluted to 500 μl with HBG or water before measurement. Results are shown in figure 4.

In vivo biocompatibility of polymers in mice

Polymers were administered by tail vein injection in 200 μL HBG (20 mM Hepes buffered 5% glucose, pH 7.1) into nude mice. In every case two doses were tested that correspond to amounts present in 50 μg siRNA polyplexes per 20 g mouse at a protonable nitrogen: phosphate charge ratio (N/P) of either 6 or 12. No visible toxic effects were observed, immediately or after 3 hours. All animals survived the observation period of one day.

Tested polymers:

| Polymer ID | Sequence |
|---|---|
| 45 | C-Stp3-C-K-MyrA2 |
| 46 | C-Stp3-C-K-OleA2 |
| 51 | C-Stp3-C-K |
| 26 | K-Stp5-K-MyrA2 |
| 27 | K-Stp5-K-OleA2 |
| 23 | K-Stp5-K |
| 49 | C-Stp2-K-(K-OleA2)-Stp2-C |
| 69 | C-Stp2-K-MyrA2 |
| 70 | C-K-Stp2-K-MyrA2 |
| 72 | C-Stp2-K-OleA2 |
| 71 | C-K-Stp2-K-OleA2 |

In vivo luciferase reporter gene expression after systemic application

Female 8-week-old A/J mice were inoculated subcutaneously in the flank with 1 million Neuro2a cells. Experiments started when tumors reached a weight of 100-750 mg. Systemic gene transfer in tumor bearing mice ( 4-5 mice per group) was carried out using polyplexes containing 50 μg pEGFPLuc DNA (Clontech Laboratories, Mountain View, CA) per 20 g body weight at a concentration of 200 μg/ml DNA in HBG and a polymer/DNA w/w ratio of 10. Polyplexes were applied into the tail vein and animals were sacrificed 24 h after application. Tissues as indicated were dissected and homogenized in cell culture lysis reagent (Promega, Mannheim, Germany) using an IKA-Ultra-Turrax homogeniser and subsequently centrifuged at 4000 rpm at 4 °C for 20 minutes to separate insoluble cell components. Luciferase activity was determined in the supernatant using a luminometer as described above. The results (luciferase gene expression per organs: He, heart; Lu, lung; Li, liver; Sp, spleen; Ki, kidney; Tu, tumor; Mu, muscle; Va, isolated blood vessel) are shown in figure 9. Results for polymers 46 , 49, 82 are shown.

## Claims

**1.** A polymer formed by 2-60 oligo(alkyleneamino) acid units of the general structure

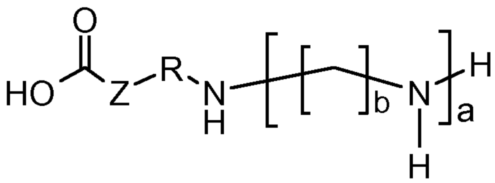

wherein

Z is chosen from either an alkyl group with the general structure

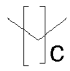,

wherein *c* is 0-8; or an amino group with the general structure

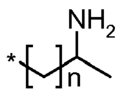,

wherein *n* is 1-7; or an aromatic group with the structure

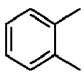, or 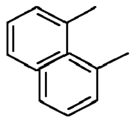;

*R* is either $CH_2$ or C=O; and

*a* is 1-7 and *b* is 2-7.

**2.** The polymer of claim 1, formed by 2-10 oligo(alkyleneamino) acid units, preferably by 2-5 oligo(alkyleneamino) acid units.

**3.** The polymer of any of claims 1 to 2 wherein Z is an alkyl group with the general structure

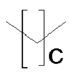,

wherein *c* is 0-8.

**4.** The polymer of any of claims 1 to 3, wherein R is C=O.

**5.** The polymer of any of claims 1 to 4, wherein b is 2 .

**6.** The polymer of any of claims 1 to 5, further comprising a hydrophobic domain covalently linked to the polymer.

**7.** The polymer of claim 6, wherein the hydrophobic domain comprises 1-10 fatty acids.

**8.** The polymer of any of claims 6 to 7, wherein the hydrophobic domain comprises 2 fatty acids.

**9.** The polymer of any of claims 6 to 8, wherein the fatty acid is chosen from the group of butyric acid, caprylic acid, myristic acid, oleic acid, arachidic acid, stearic acid, lauric acid and palmitic acid.

**10.** The polymer of any of claims 6 to 9, wherein the hydrophobic domain is covalently linked to an amine group at the N-terminus of the polymer.

**11.** The polymer of any of claims 6 to 10, wherein the hydrophobic domain is covalently linked to an amine group at the N-terminus of the polymer via a linker moiety, wherein the linker moiety is an amino acid.

**12.** The polymer of any of claims 1 to 5, wherein the polymer is branched.

**13.** The branched polymer of claim 12, wherein two polymer chains according to claims 1 to 5 are connected via a linker moiety, wherein the linker moiety is an amino acid.

**14.** The branched polymer of claim 12, further comprising a hydrophobic domain according to claims 7-9 covalently linked to the amine group of the linker moiety.

**15.** The polymer of any of claims 1 to 14, further comprising a headgroup covalently linked to one terminus of the polymer.

**16.** The polymer of any of claims 1 to 15, wherein the headgroup is covalently linked to the carboxyl group at the C-terminus.

**17.** The polymer of any of claims 1 to 16 wherein the headgroup is a polar positively charged head group.

**18.** The polymer of any of claims 1 to 17 wherein the polar positively charged head group is chosen from cysteine, lysine, arginine, histidine or a combination thereof.

**19.** The polymer of any of claims 1 to 18 wherein the headgroup is a coupling or crosslinking domain.

**20.** The polymer of any of claims 1 to 19 wherein the coupling or crosslinking domain comprises a cysteine group.

**21.** The polymer of any of claims 1 to 20, further comprising a peptide sequence.

**22.** The polymer of any of claims 1 to 21, further comprising a shielding moiety.

**23.** The polymer of any of claims 1 to 22, wherein the polymer is complexed with a nucleic acid.

**24.** The polymer of claim 23, wherein the nucleic acid is a siRNA.

**25.** Use of the polymer of any of claims 1 to 24 for the delivery of nucleic acids.

**26.** A composition comprising the polymer of any of claims 1 to 22 and a nucleic acid.

Fig. 1

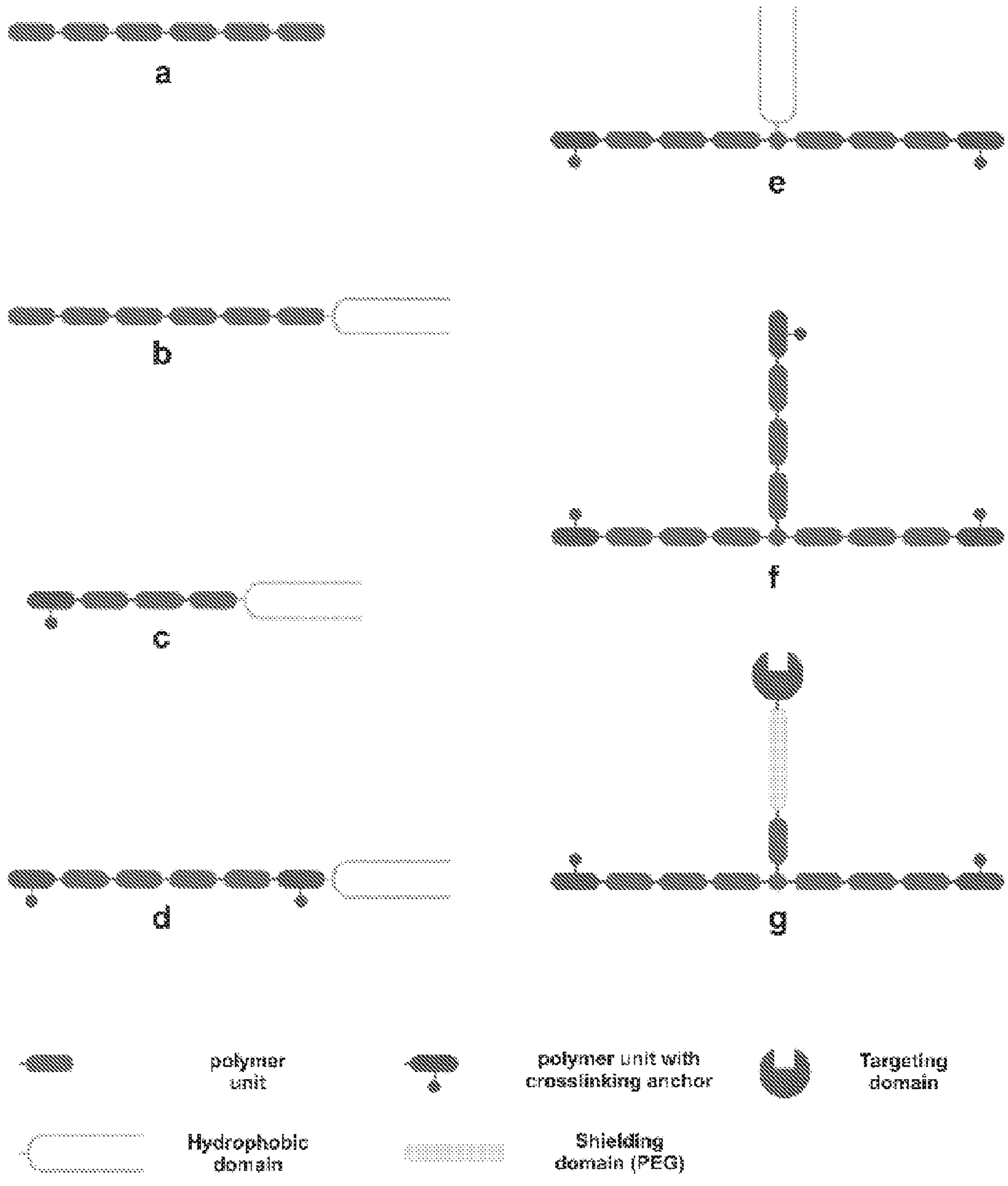
a
b
c
d
e
f
g
polymer unit
polymer unit with crosslinking anchor
Targeting domain
Hydrophobic domain
Shielding domain (PEG)

Fig. 2

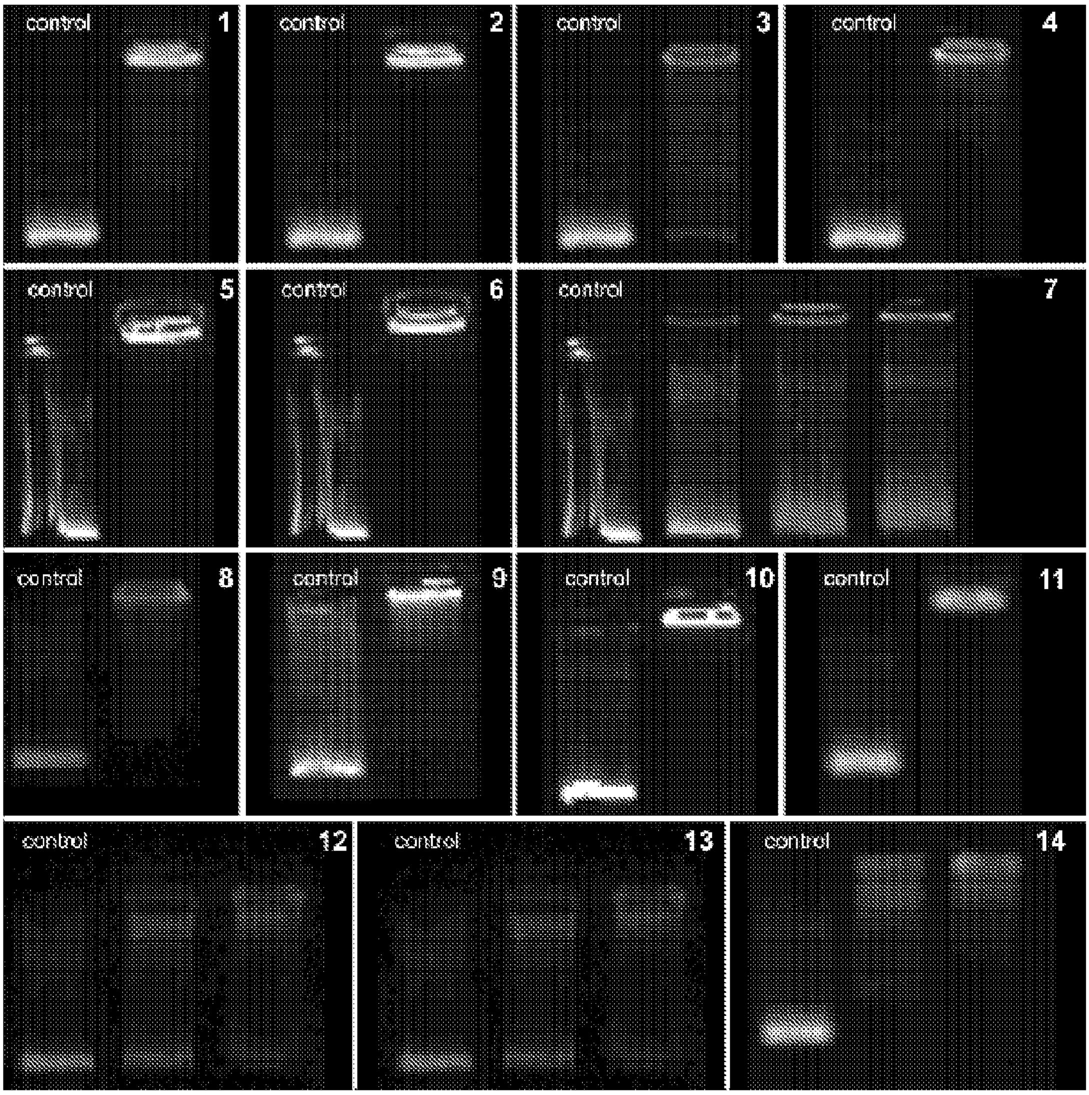
control
1
control
2
control
3
control
4
control
5
control
6
control
7
control
8
control
9
control
10
control
11
control
12
control
13
control
14

Fig. 3

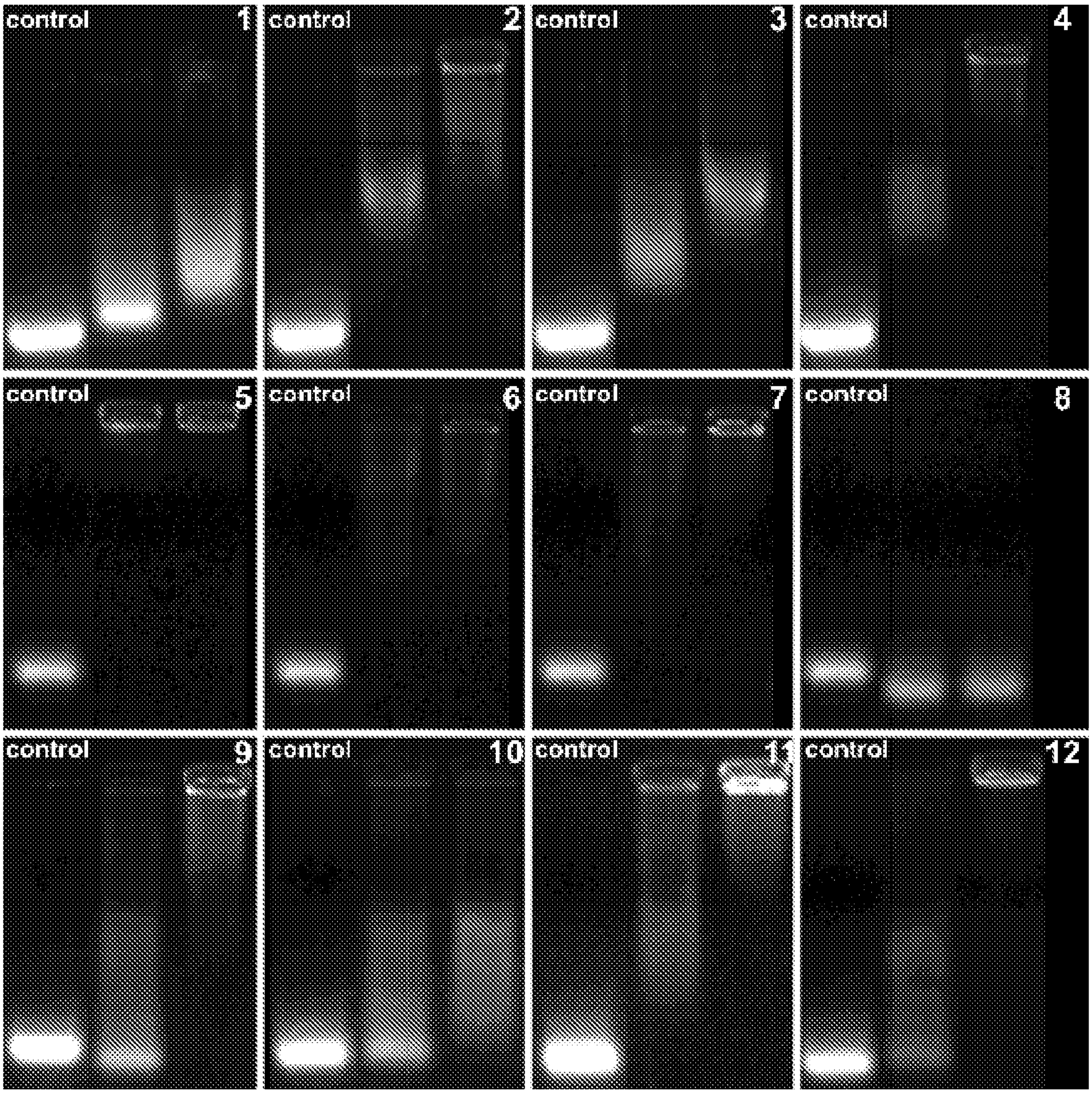
control
1
control
2
control
3
control
4
control
5
control
6
control
7
control
8
control
9
control
10
control
11
control
12

**Fig. 4**

| | DNA conc [µg/ml] | w/w ratio | Size [nm] | Zeta* [mV] |
|---|---|---|---|---|
| 46 | 100 | 5 | 92,3 | 18,1 |
| | 100 | 10 | 159,4 | - |
| | 200 | 5 | 7185 | 19,4 |
| | 200 | 10 | 218,4 | - |
| 49 | 100 | 5 | 171,3 | 26,0 |
| | 100 | 10 | 150,3 | - |
| | 200 | 5 | 197,2 | 24,1 |
| | 200 | 10 | 173,3 | - |

| | siRNA conc [µg/ml] | N/P ratio | Size [nm] | Zeta [mV] |
|---|---|---|---|---|
| 46 | 100 | 6 | 7417 | 8,8 |
| | 200 | 6 | 215,7 | 14,5 |
| 49 | 100 | 6 | 198 | 21,3 |
| | 200 | 6 | 236 | 24,3 |

Fig. 5

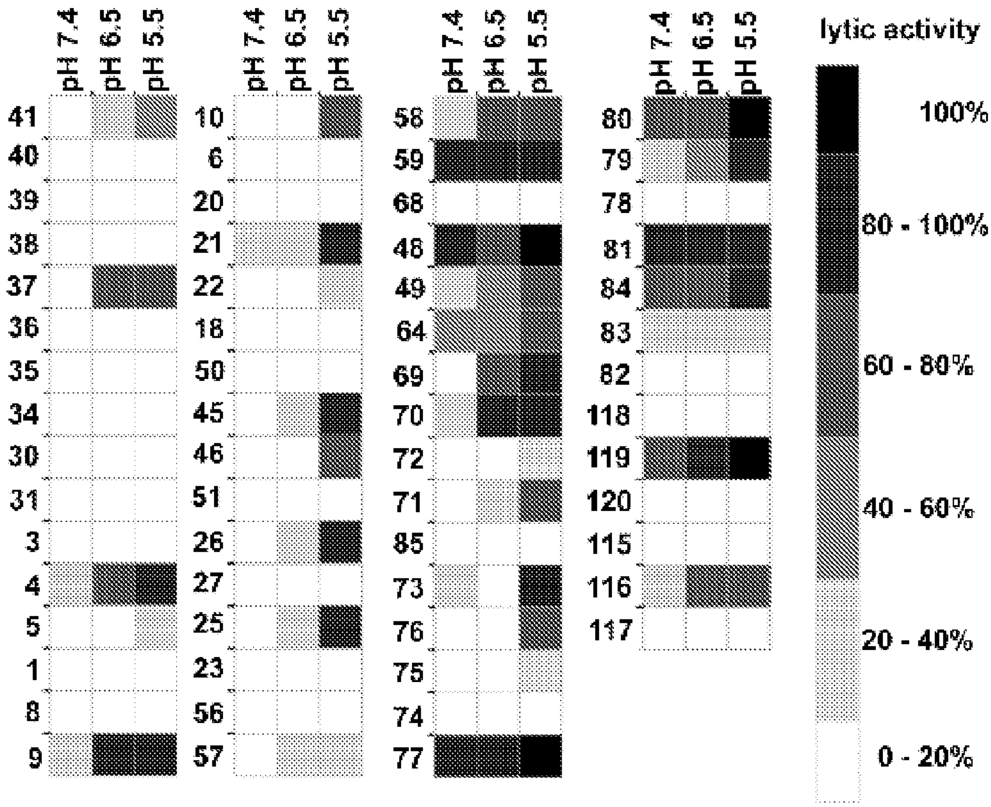
pH 7.4
pH 6.5
pH 5.5
41
40
39
38
37
36
35
34
30
31
3
4
5
1
8
9
10
6
20
21
22
18
50
45
46
51
26
27
25
23
56
57
58
59
68
48
49
64
69
70
72
71
85
73
76
75
74
77
80
79
78
81
84
83
82
118
119
120
115
116
117
lytic activity
100%
80 - 100%
60 - 80%
40 - 60%
20 - 40%
0 - 20%

Fig. 6 a)

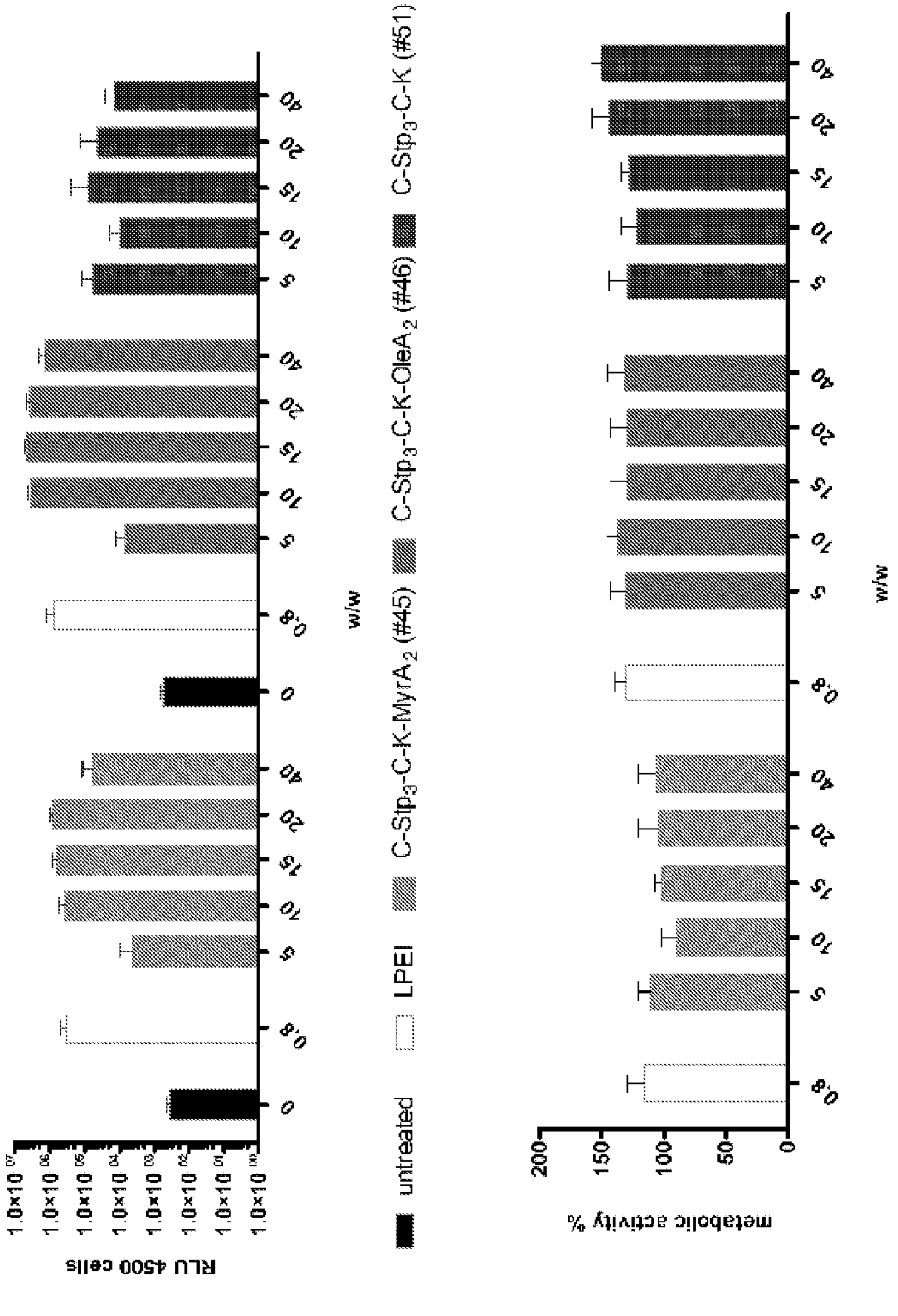
RLU 4500 cells
untreated
LPEI
C-Stp$_3$-C-K-MyrA$_2$ (#45)
C-Stp$_3$-C-K-OleA$_2$ (#46)
C-Stp$_3$-C-K (#51)
w/w
metabolic activity %

Fig. 6b)

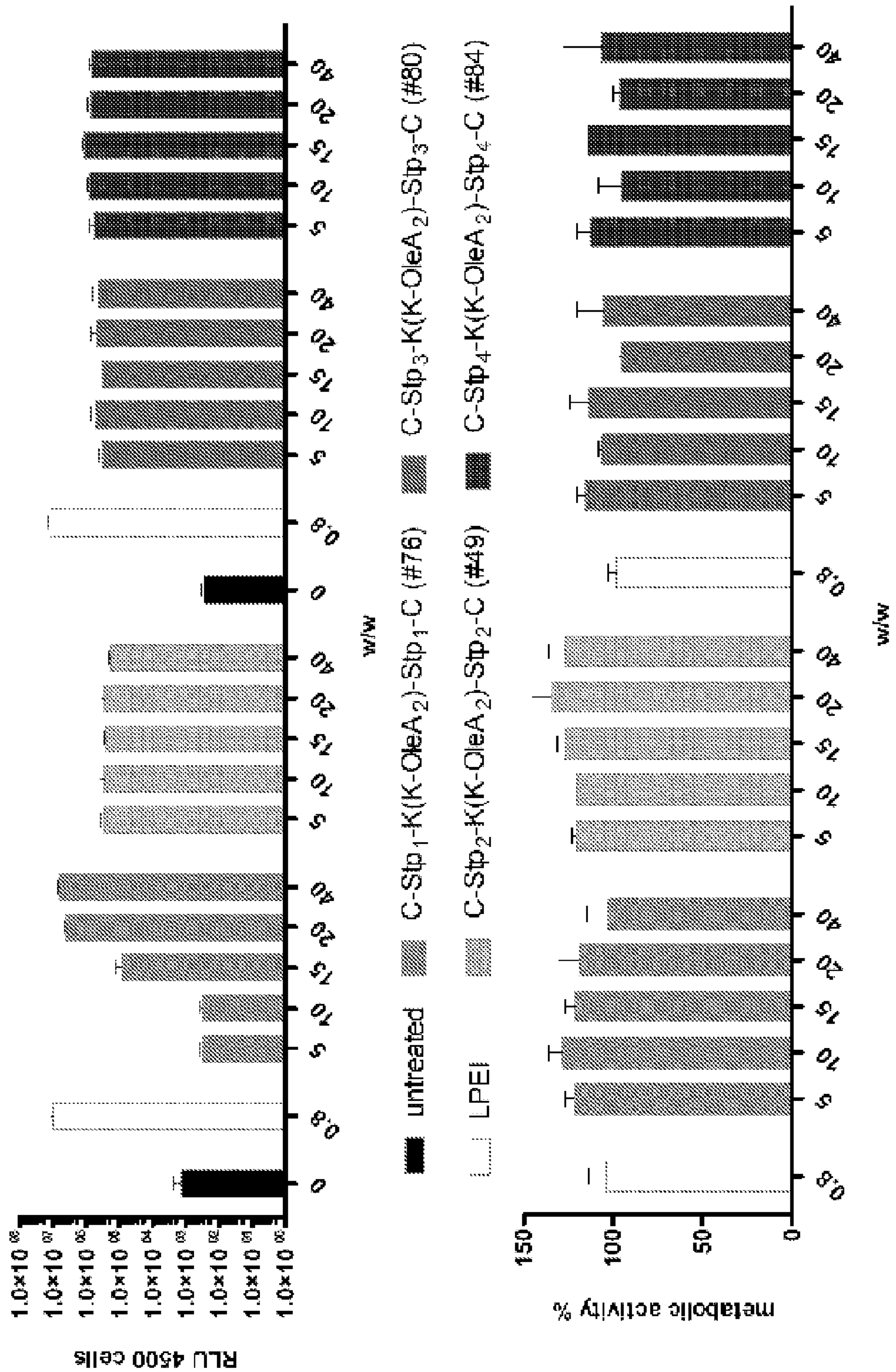
RLU 4500 cells
w/w
metabolic activity %
w/w
untreated
LPEI
C-Stp$_1$-K(K-OleA$_2$)-Stp$_1$-C (#76)
C-Stp$_2$-K(K-OleA$_2$)-Stp$_2$-C (#49)
C-Stp$_3$-K(K-OleA$_2$)-Stp$_3$-C (#80)
C-Stp$_4$-K(K-OleA$_2$)-Stp$_4$-C (#84)

Fig. 6c)

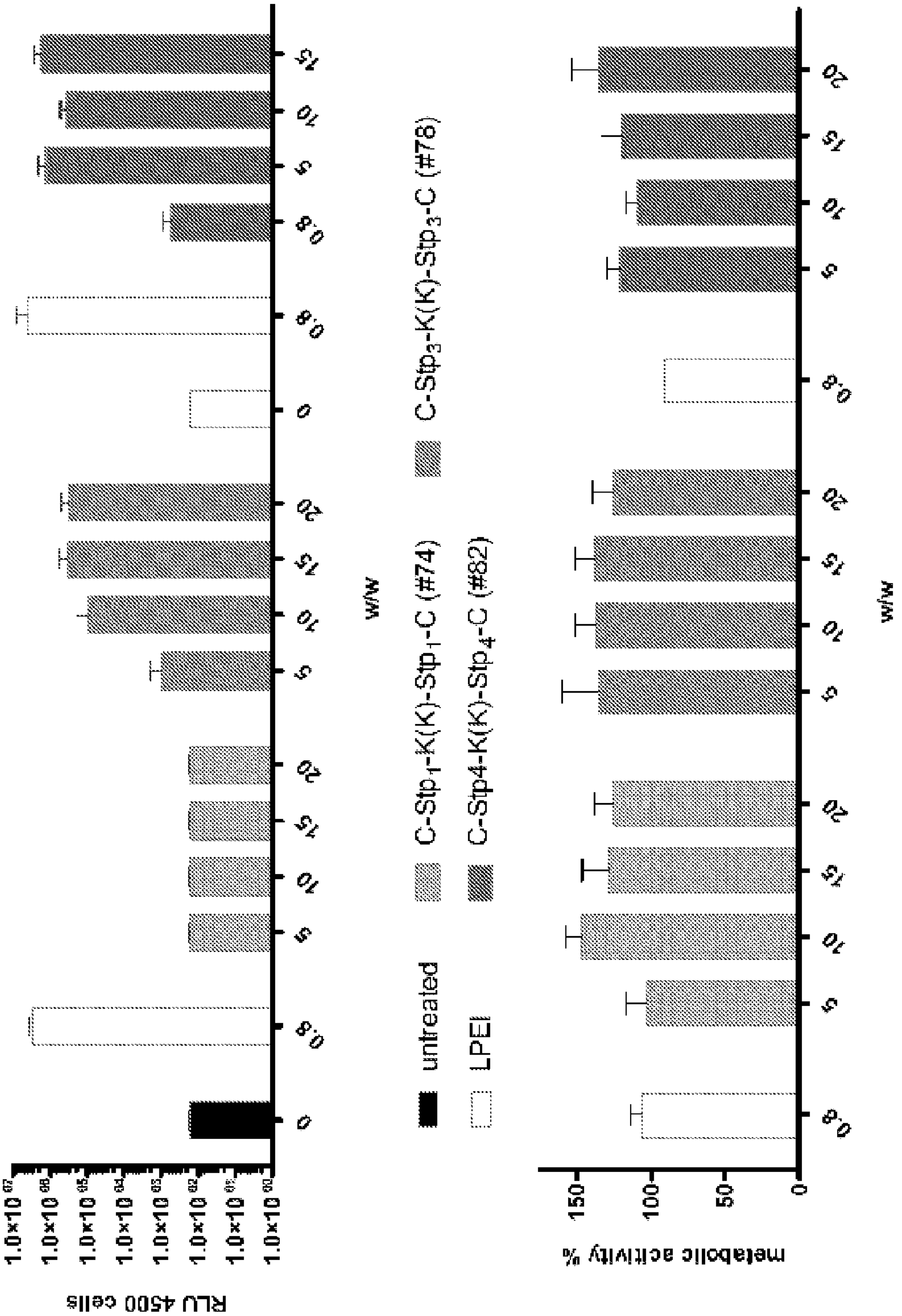
RLU 4500 cells
metabolic activity %
w/w
untreated
LPEI
C-Stp$_1$-K(K)-Stp$_1$-C (#74)
C-Stp4-K(K)-Stp$_4$-C (#82)
C-Stp$_3$-K(K)-Stp$_3$-C (#78)

**Fig. 7** a)

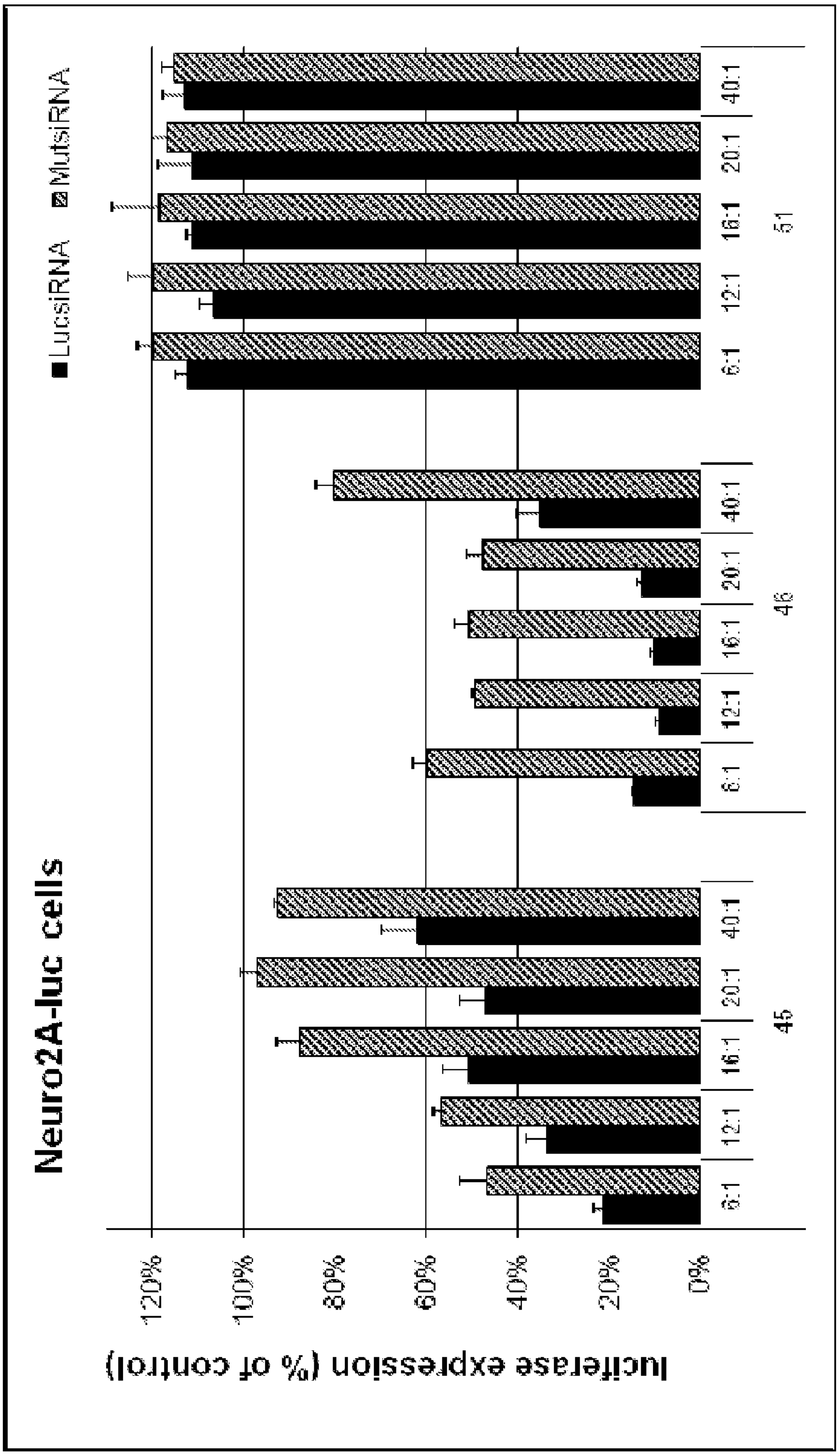
Neuro2A-luc cells
LucsiRNA
MutsiRNA
luciferase expression (% of control)
120%
100%
80%
60%
40%
20%
0%
6:1
12:1
16:1
20:1
40:1
45
46
51

Fig. 7 b)

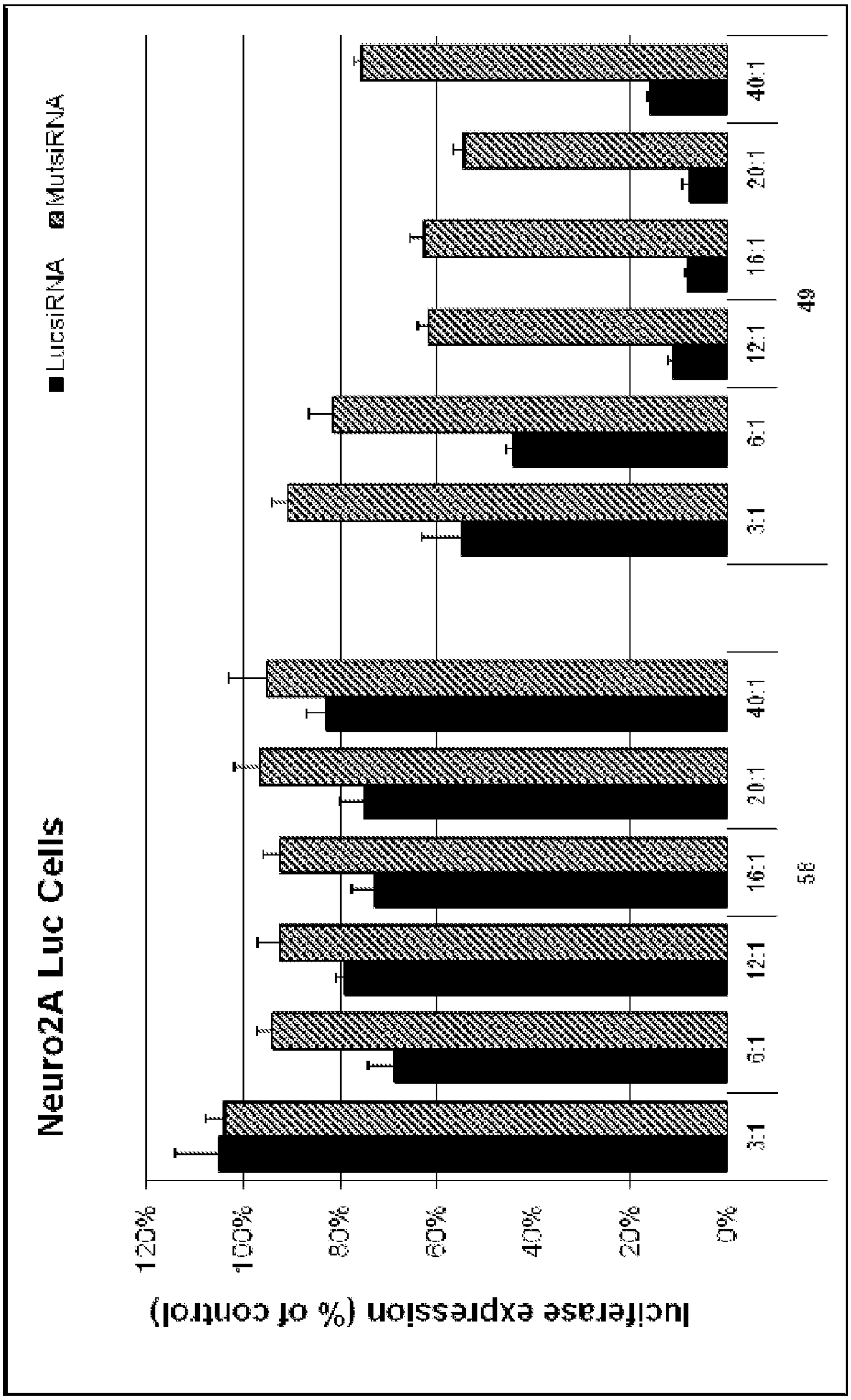
Neuro2A Luc Cells
LucsiRNA
MutsiRNA
luciferase expression (% of control)
120%
100%
80%
60%
40%
20%
0%
3:1
6:1
12:1
16:1
20:1
40:1
58
3:1
6:1
12:1
16:1
20:1
40:1
49

**Fig. 7 c)**

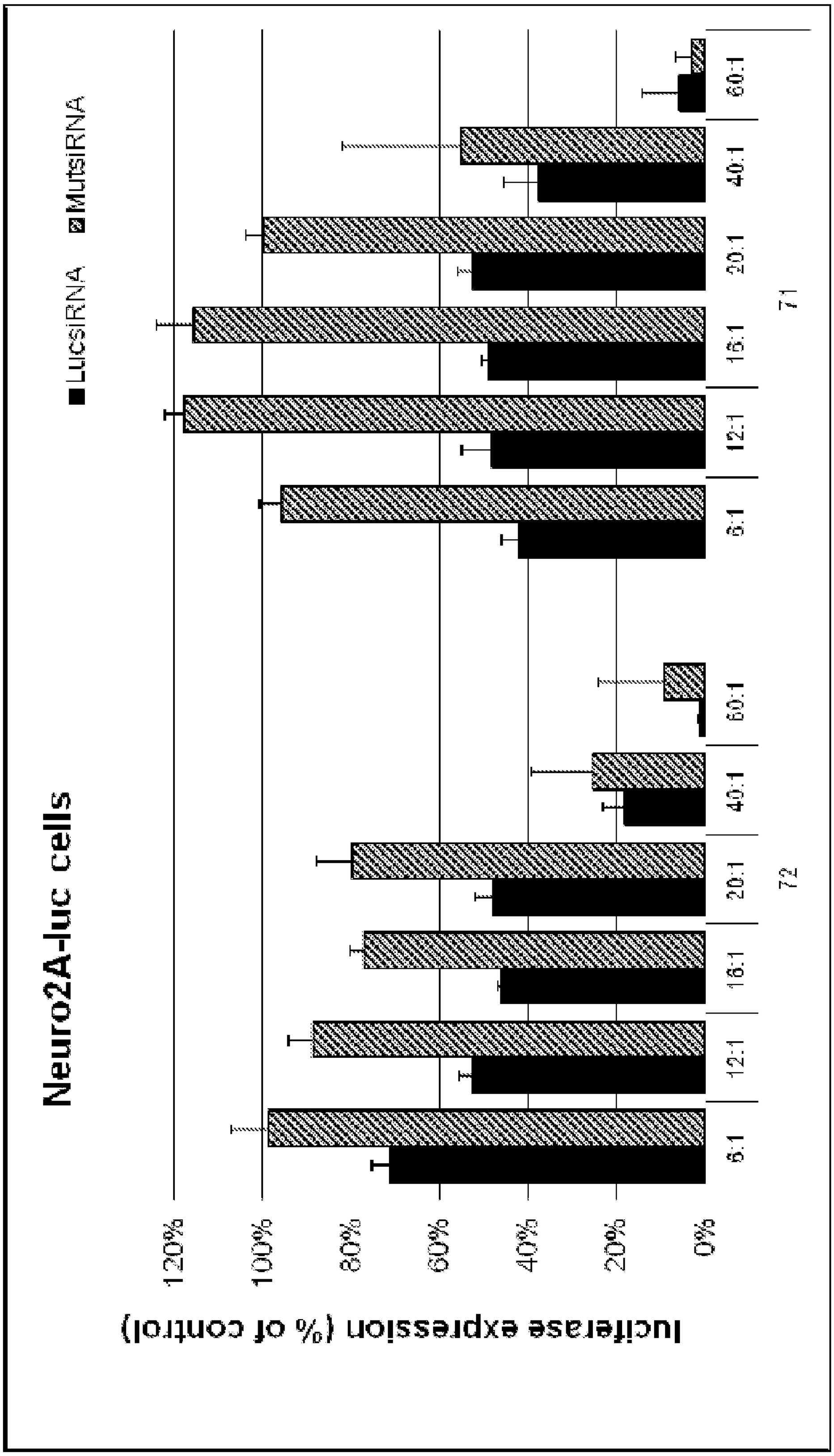
Neuro2A-luc cells
LucsiRNA
MutsiRNA
luciferase expression (% of control)
120%
100%
80%
60%
40%
20%
0%
6:1
12:1
16:1
20:1
40:1
60:1
72
6:1
12:1
16:1
20:1
40:1
60:1
71

**Fig. 7 d)**

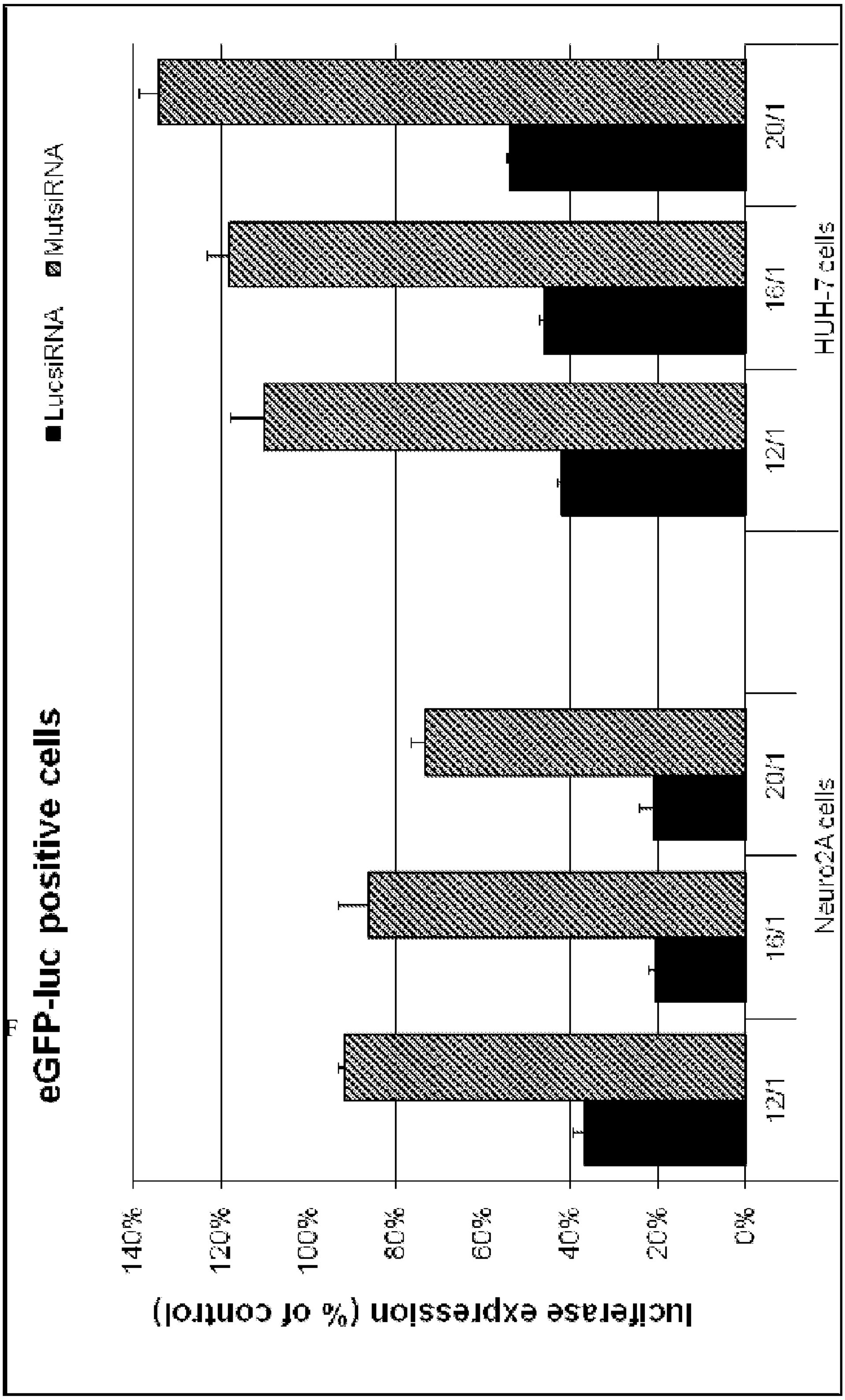
eGFP-luc positive cells
LucsiRNA
MutsiRNA
luciferase expression (% of control)
140%
120%
100%
80%
60%
40%
20%
0%
12/1
16/1
20/1
Neuro2A cells
12/1
16/1
20/1
HUH-7 cells

Fig. 7e)

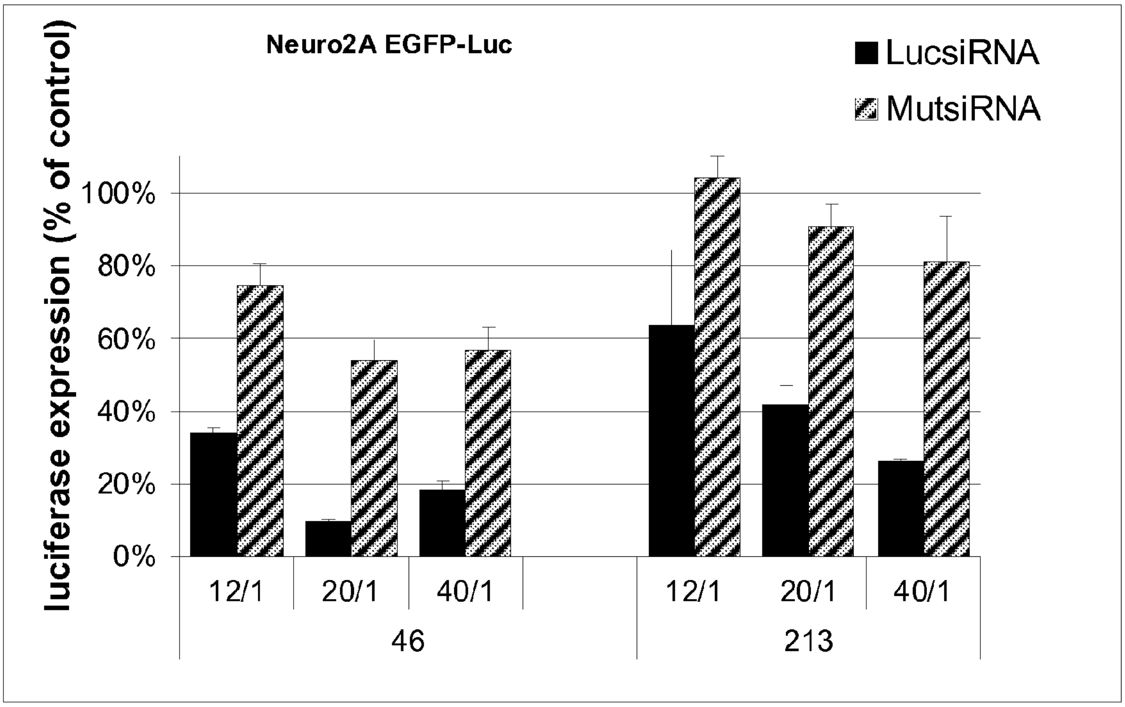
Neuro2A EGFP-Luc
LucsiRNA
MutsiRNA
luciferase expression (% of control)
100%
80%
60%
40%
20%
0%
12/1
20/1
40/1
12/1
20/1
40/1
46
213

**Fig. 8** a)

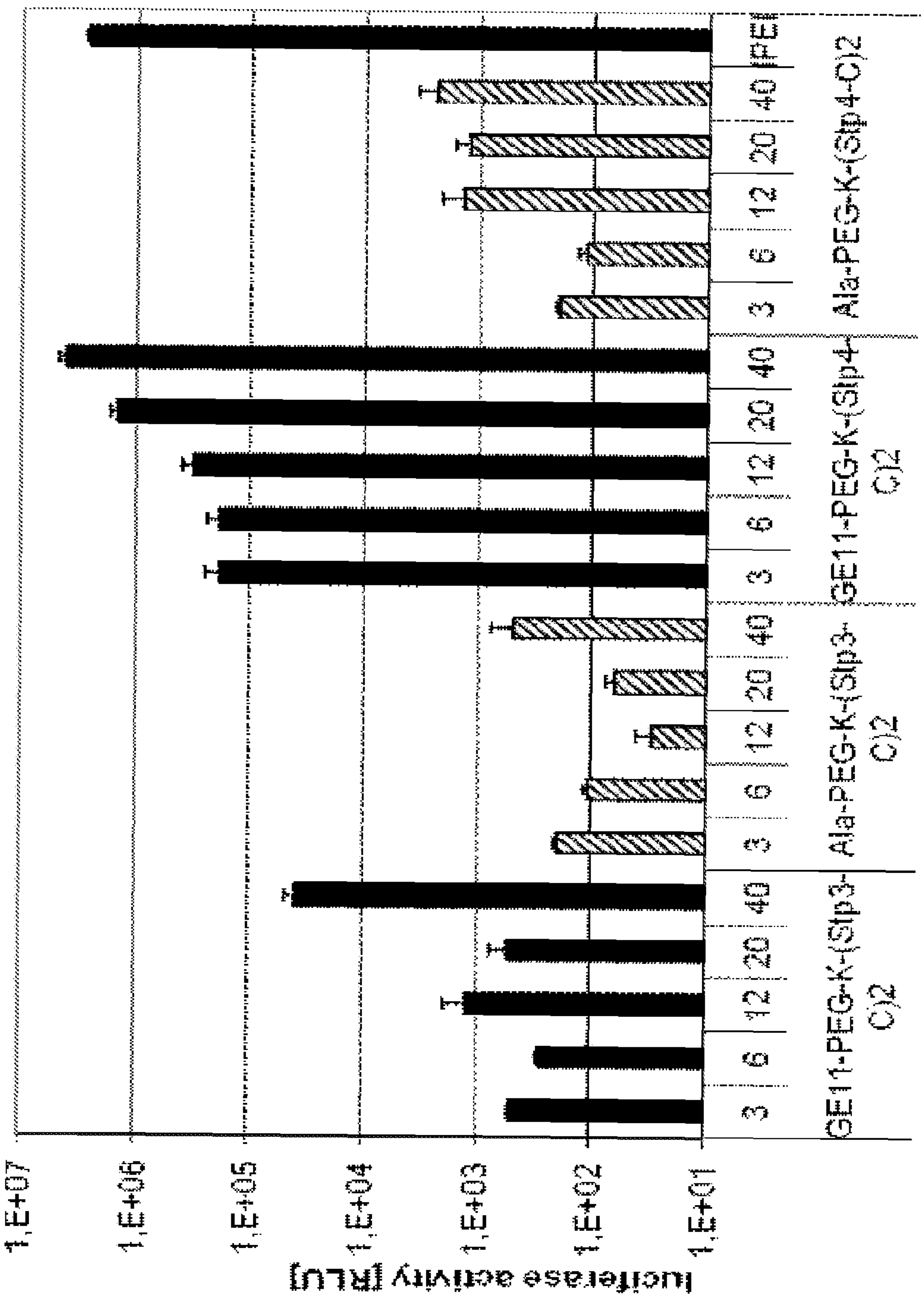
luciferase activity [RLU]
1,E+07
1,E+06
1,E+05
1,E+04
1,E+03
1,E+02
1,E+01
3 6 12 20 40
GE11-PEG-K-(Stp3-C)2
Ala-PEG-K-(Stp3-C)2
GE11-PEG-K-(Stp4-C)2
Ala-PEG-K-(Stp4-C)2
PEI
N / P

Fig. 8 b)

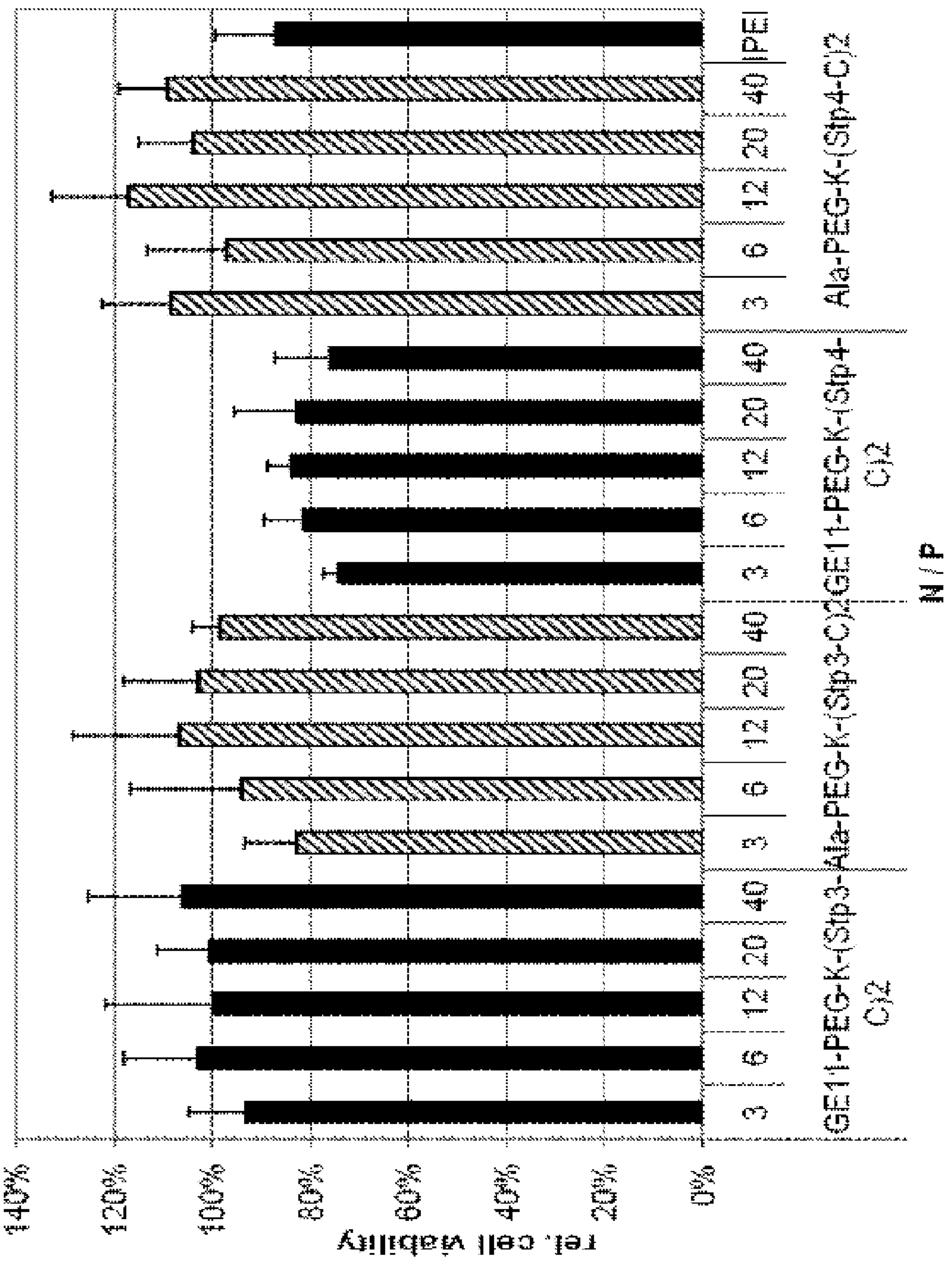
rel. cell viability
0%
20%
40%
60%
80%
100%
120%
140%
N / P
GE11-PEG-K-(Stp3-C)2
Ala-PEG-K-(Stp3-C)2
GE11-PEG-K-(Stp4-C)2
Ala-PEG-K-(Stp4-C)2
PEI
3
6
12
20
40

Fig. 9 a)

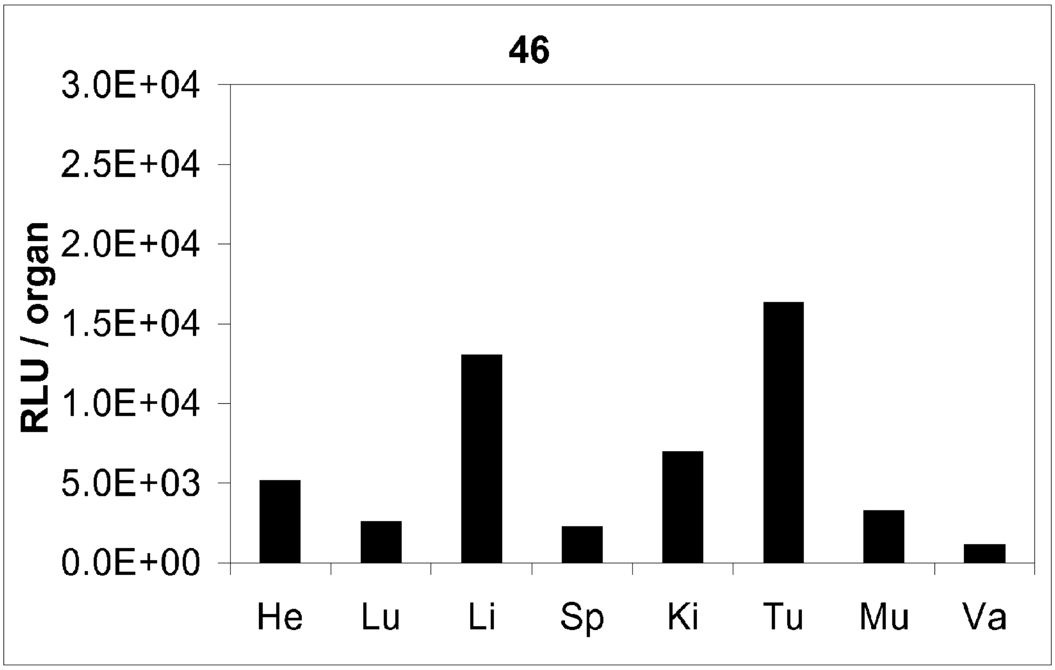
46
RLU / organ
3.0E+04
2.5E+04
2.0E+04
1.5E+04
1.0E+04
5.0E+03
0.0E+00
He
Lu
Li
Sp
Ki
Tu
Mu
Va

Fig. 9 b)

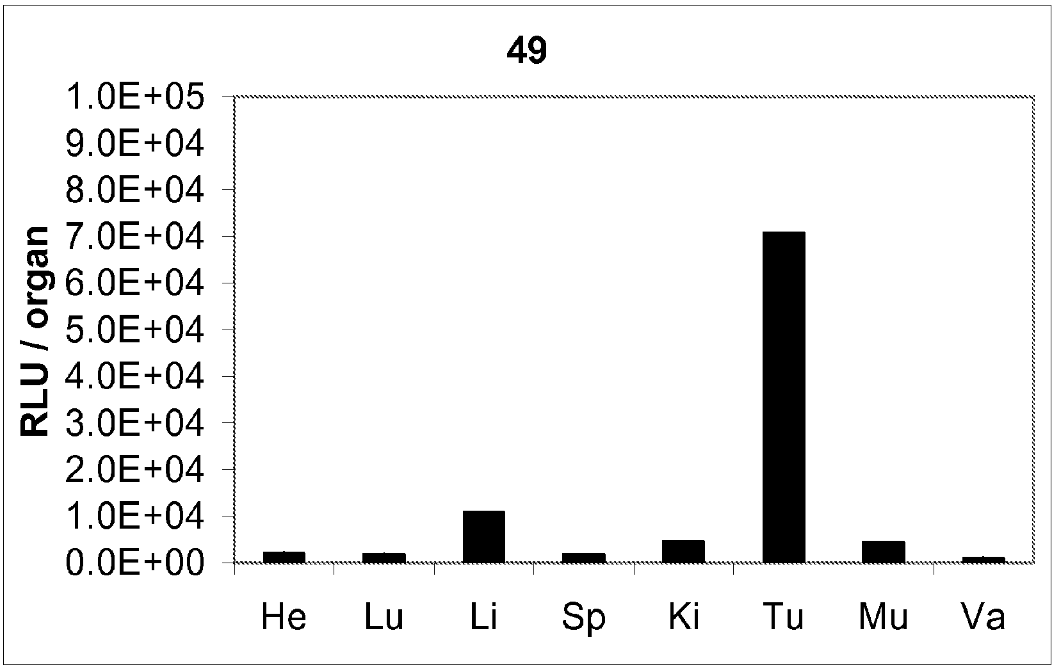
49
1.0E+05
9.0E+04
8.0E+04
7.0E+04
6.0E+04
5.0E+04
4.0E+04
3.0E+04
2.0E+04
1.0E+04
0.0E+00
RLU / organ
He
Lu
Li
Sp
Ki
Tu
Mu
Va

Fig. 9 c)

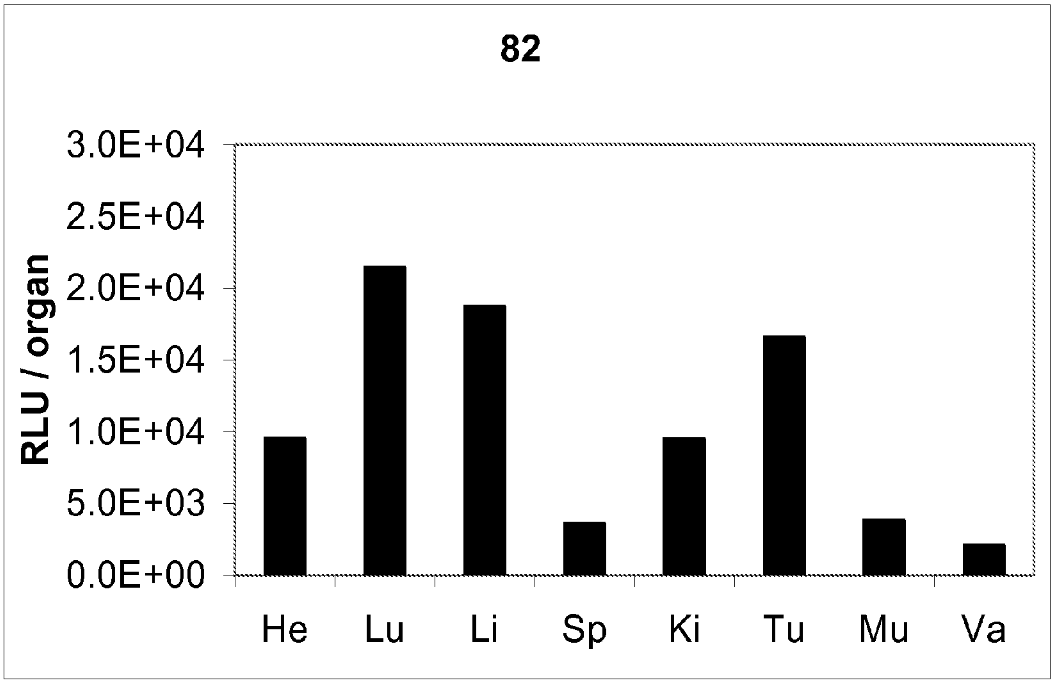
82
3.0E+04
2.5E+04
2.0E+04
1.5E+04
1.0E+04
5.0E+03
0.0E+00
RLU / organ
He
Lu
Li
Sp
Ki
Tu
Mu
Va

Europäisches Patentamt
European Patent Office
Office européen des brevets

# EUROPEAN SEARCH REPORT

Application Number
EP 10 16 5502

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D<br><br><br>A | EP 1 400 551 A1 (GRYPHON SCIENCES [US] AMYLIN PHARMACEUTICALS INC [US]) 24 March 2004 (2004-03-24)<br>* page 22, line 36 - line 37 *<br>* page 23, line 1 *<br>* page 28; example 9 *<br>----- | 1-25<br><br><br>26 | INV.<br>C08G73/02<br>C08G69/26<br>C08G69/08<br>A61K47/34<br>C12N15/113<br>A61K48/00 |
| X | LAURA HARTMANN, STEFANIE HÄFELE, REGINE PESCHKA-SÜSS, MARKUS ANTONIETTI, HANS G. BÖRNER: "Tailor-Made Poly(amidoamine)s for Controlled Complexation and Condensation of DNA"<br>CHEM. EUR. J.,<br>vol. 14, no. 7,<br>7 February 2008 (2008-02-07), pages 2025-2033, XP002595989 DOI: 10.1002/chem.200701223<br>* page 2027, scheme 1, PEO-spPAA (III) *<br>----- | 1-26 | |
| X<br><br><br><br><br><br><br>A | SABRINA CARROCCIO, CONCETTO PUGLISI, GIORGIO MONTAUDO: "MALDI Investigation of the Photooxidation of Nylon-66"<br>MACROMOLECULES,<br>vol. 37, no. 16, 14 July 2004 (2004-07-14), pages 6037-6049, XP002595990 DOI: 10.1021/ma049521n<br>* page 6041, table 2, oligomers with n=9 *<br>----- | 1-24<br><br><br><br><br><br><br>25,26 | TECHNICAL FIELDS SEARCHED (IPC)<br>C08G<br>A61K<br>C12N |
| X<br><br>A | US 2004/198947 A1 (MASLANKA WILLIAM W [US]) 7 October 2004 (2004-10-07)<br>* example 4 *<br>----- | 1-24<br><br>25,26 | |
| X<br><br>A | GB 1 509 967 A (BASF AG)<br>10 May 1978 (1978-05-10)<br>* pages 5-6, "(B) Manufacture of polyamidoamines V6" *<br>-----<br>-/-- | 1-24<br><br>25,26 | |

1

The present search report has been drawn up for all claims

| Place of search | Date of completion of the search | Examiner |
|---|---|---|
| Munich | 8 November 2010 | Barrère, Matthieu |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 10 16 5502

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (IPC) |
| A | PAOLO FERRUTI ET AL: "POLY(AMIDO-AMINE)S: BIOMEDICAL APPLICATIONS" MACROMOLECULAR: RAPID COMMUNICATIONS, WILEY VCH VERLAG, WEINHEIM, DE LNKD- DOI:10.1002/1521-3927(20020401)23:5/6&LT,332::AID-MARC332&GT,3.0.CO,2-I, vol. 23, 1 January 2002 (2002-01-01), pages 332-355, XP008077050 ISSN: 1022-1336 * abstract * * pages 349-353, paragraph 4.4.3 * ----- | 1-26 | |
| | | | TECHNICAL FIELDS SEARCHED (IPC) |

The present search report has been drawn up for all claims

| Place of search | Date of completion of the search | Examiner |
|---|---|---|
| Munich | 8 November 2010 | Barrère, Matthieu |

1

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 10 16 5502

This annex lists the patent family members relating to the patent documents cited in the above-mentioned European search report.
The members are as contained in the European Patent Office EDP file on
The European Patent Office is in no way liable for these particulars which are merely given for the purpose of information.

08-11-2010

| Patent document cited in search report | | Publication date | Patent family member(s) | | | Publication date |
|---|---|---|---|---|---|---|
| EP 1400551 | A1 | 24-03-2004 | NONE | | | |
| US 2004198947 | A1 | 07-10-2004 | AU | 2004230842 | A1 | 28-10-2004 |
| | | | BR | PI0408995 | A | 28-03-2006 |
| | | | CA | 2517592 | A1 | 28-10-2004 |
| | | | CN | 1771279 | A | 10-05-2006 |
| | | | EP | 1611185 | A1 | 04-01-2006 |
| | | | JP | 2006522208 | T | 28-09-2006 |
| | | | KR | 20050114697 | A | 06-12-2005 |
| | | | MX | PA05009826 | A | 05-12-2005 |
| | | | WO | 2004092248 | A1 | 28-10-2004 |
| | | | ZA | 200508857 | A | 29-11-2006 |
| GB 1509967 | A | 10-05-1978 | AT | 350272 | B | 25-05-1979 |
| | | | AU | 8280075 | A | 13-01-1977 |
| | | | BE | 831481 | A1 | 19-01-1976 |
| | | | CA | 1058794 | A1 | 17-07-1979 |
| | | | CH | 616170 | A5 | 14-03-1980 |
| | | | DE | 2434816 | A1 | 05-02-1976 |
| | | | DE | 2515760 | A1 | 21-10-1976 |
| | | | FI | 752009 | A | 20-01-1976 |
| | | | FR | 2330799 | A1 | 03-06-1977 |
| | | | JP | 1121468 | C | 12-11-1982 |
| | | | JP | 51084895 | A | 24-07-1976 |
| | | | JP | 57005813 | B | 02-02-1982 |
| | | | NL | 7508636 | A | 21-01-1976 |
| | | | NO | 752474 | A | 20-01-1976 |
| | | | SE | 450958 | B | 17-08-1987 |
| | | | SE | 7508201 | A | 20-01-1976 |



For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5948902 A


### Non-patent literature cited in the description


- **HARTMANN L ; HAFELE S ; PESCHKA-SUSS R ; ANTONIETTI M ; BOMER HG.** Tailor-Made Poly(amidoamine)s for Controlled Complexation and Condensation of DNA. *Chemistry,* 2008, vol. 14 (7), 2025-2033
- **WANG XL ; RAMUSOVIC S ; NGUYEN T ; LU ZR.** Novel polymerizable surfactants with pH-sensitive amphiphilicity and cell menrane disruption for efficient siRNA delivery. *Bioconjugate Chemistry,* 2007, vol. 18, 2169-2177
- **ZOU SM ; ERBACHER P ; REMY JS ; BEHR JP.** *J Gene Med.,* March 2000, vol. 2 (2), 128-34

SEQUENCE LISTING

<110> F. Hoffmann-La Roche AG / LMU München

<120> Polymers for delivery of nucleic acids

<130> 26558

<160> 8

<170> PatentIn version 3.5

<210> 1
<211> 21
<212> DNA
<213> Artificial Sequence

<220>
<223> luciferase-siRNA GL3 luciferase duplex, sense strand

<400> 1
cuuacgcuga guacuucgat t 21

<210> 2
<211> 21
<212> DNA
<213> Artificial Sequence

<220>
<223> luciferase-siRNA GL3 luciferase duplex, antisense strand

<400> 2
ucgaaguacu cagcguaagt t 21

<210> 3
<211> 21
<212> DNA
<213> Artificial Sequence

<220>
<223> control-siRNA (non-targeting control duplex), sense strand

<400> 3
auguauuggc cuguauuagu u 21

<210> 4
<211> 21
<212> DNA
<213> Artificial Sequence

<220>
<223> control-siRNA (non-targeting control duplex), antisense strand

<400> 4
cuaauacagg ccaauacauu u 21

<210> 5
<211> 21
<212> DNA

```
<213>  Artificial Sequence

<220>
<223>  EGFPLuc-siRNA duplex sense strand

<400>  5
auaucauggc cgacaagcat t                                                  21


<210>  6
<211>  21
<212>  DNA
<213>  Artificial Sequence

<220>
<223>  EGFPLuc-siRNA duplex antisense

<400>  6
ugcuugucgg ccaugauaut t                                                  21


<210>  7
<211>  21
<212>  DNA
<213>  Artificial Sequence

<220>
<223>  Control siRNA duplex sense strand

<400>  7
auguauuggc cuguauuagt t                                                  21


<210>  8
<211>  21
<212>  DNA
<213>  Artificial Sequence

<220>
<223>  Control siRNA duplex antisense strand

<400>  8
cuaauacagg ccaauacaut t                                                  21




2
```